(19)

Europäisches
Patentamt
European
Patent Office
Office européen
des brevets

(11) **EP 2 338 529 B1**

(12) **EUROPÄISCHE PATENTSCHRIFT**

(45) Veröffentlichungstag und Bekanntmachung des
Hinweises auf die Patenterteilung:
**29.05.2013 Patentblatt 2013/22**

(51) Int Cl.:
*A61L 15/26* ^(2006.01)          *A61L 15/60* ^(2006.01)

(21) Anmeldenummer: **09016007.8**

(22) Anmeldetag: **24.12.2009**

(54) **Hydrogelmatrix mit verbesserten Klebeeigenschaften**

Hydrogel matrix with improved adhesive characteristics

Matrice d'hydrogel dotée de propriétés d'adhésion améliorées

(84) Benannte Vertragsstaaten:
**AT BE BG CH CY CZ DE DK EE ES FI FR GB GR
HR HU IE IS IT LI LT LU LV MC MK MT NL NO PL
PT RO SE SI SK SM TR**

(43) Veröffentlichungstag der Anmeldung:
**29.06.2011 Patentblatt 2011/26**

(73) Patentinhaber: **Paul Hartmann AG
89522 Heidenheim (DE)**

(72) Erfinder:
• **Junginger, Martin
89568 Hermaringen (DE)**
• **Horny, Julie
68540 Bollwiller (FR)**

(56) Entgegenhaltungen:
EP-A1- 0 528 091          WO-A1-02/45761
DE-A1-102005 027 656      DE-A1-102006 016 636
US-A- 5 292 777           US-A1- 2006 200 063

EP 2 338 529 B1

**Beschreibung**

**Beschreibung**

Die Erfindung betrifft Wundauflagen insbesondere als Wundbehandlungsmittel in der Granulations- und Epithelisierungsphase. Diese Wundauflagen können insbesondere zur feuchten Wundbehandlung eingesetzt werden.

Die Heilung von Hautwunden beruht auf der Fähigkeit der Haut Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).

Zur Unterstützung der einzelnen Wundheilungsphasen sind zahlreiche Vorschläge in der Literatur beschrieben. Insbesondere sind auch Wundauflagen mit Hydrogelen seit geraumer Zeit Gegenstand zahlreicher Artikel in der Fachliteratur als auch von Patentliteratur. So werden beispielsweise mit den europäischen Patenten EP 455 324 B1, EP 528 091 B1, EP 567 704 B1 oder EP 630 629 B1 transparente Hydrogel-Wundauflagen mit verschiedenen Konstruktionen beschrieben. Diese zum Teil vielschichtigen Wundauflagen umfassen ein wasserhaltiges oder dehydratisiertes Hydrogel als Wundkontaktschicht zur Behandlung von Brandwunden.

Die EP0426422B1 offenbart einen Wundverband mit einem Hydrogel auf der Basis eines Polyharnstoff/Polyurethan-Copolymers. Die beschriebenen Zusammensetzungen enthalten 15 bis 30 Gew.-% eines mehrwertigen Alkohols. Das Verhältnis der reaktiven Gruppen von Isocyanat zu den Amingruppen beträgt bei den in der EP0426422B1 beschriebenen Hydrogel-Zusammensetzungen ca. 0,63 bis 1,40. Gemäß einer als bevorzugt bezeichneten Ausführungsform beträgt das Verhältnis 1,23. Eine Wundauflage, welche ein Hydrogel gemäß den in der EP0426422B1 beschriebenen Zusammensetzungen enthält, ist unter der Bezeichnung Hydrosorb® (Paul Hartmann Ag, Deutschland) auf dem Markt. Die Wundauflage kann nach einem Zeitraum von 48 Stunden etwa die doppelte Menge des Eigengewichtes an Flüssigkeit absorbieren.

Weiterhin werden beispielsweise mit den europäischen Patenten EP 457 977 B1, EP 486 522 B1, EP 541 390 B1, EP 541 391 B1, EP 570 430 B1, EP 665 856 B1, EP 691 113 B1, EP 693 913 B1 oder EP 1 082 146 B1 Wundauflagen mit verschiedenen Konstruktionen beschrieben, die als absorbierende Schicht einen Polyurethanschaum umfassen.

Darüber hinaus sind mit den europäischen Patenten EP 855 921 B1 und EP 1 156 838 B1 Wundauflagen bekannt, die einen Polyurethanschaum umfassen, der mit einem hydrophoben Silikongel beschichtet ist. Dieses Silikongel soll die Verklebung der Wunde mit dem Polyurethanschaum verhindern.

Weiterhin werden mit den internationalen Anmeldungen WO 02/ 38 097 A1, WO 02/ 47 761 A1, WO 03/ 011 352 A1, WO 03/ 086 255 A1, WO 2004/ 052 415 A1 oder EP 1 658 865 A1 Wundauflagen beschrieben, die ein Hydrogel und einen Polymerschaum umfassen.

In der Patentanmeldung des Anmelders, Anmeldenummer DE102008031183.9, welche Stand der Technik im Sinne von Artikel 54(3) EPÜ darstellt, ist gleichfalls eine mehrschichtige bzw. mehrlagige Wundauflage mit einer Wundkontaktschicht als erster Schicht und mindestens einer zweiten Schicht als absorbierende Schicht, die einen hydrophilen Polyurethanschaum umfasst, beschrieben. Die Wundkontaktschicht kann ein Hydrogel auf Basis eines Polyharnstoff/Polyurethan-Copolymers sein. Das Ausführungsbeispiel der DE102008031183.9 offenbart ein Hydrogel, welches 17,5 Gew.-% Propylenglykol umfasst, wobei das Verhältnis der reaktiven Gruppen von Isocyanat zu den Amingruppen in beschriebenen Hydrogel 1,23 beträgt. Durch die Verwendung der beschriebenen Polyharnstoff/Polyurethan-Copolymere als Wundkontaktschicht in mehrlagigen Verbänden können atraumatische Wundauflagen mit sehr guten Wundheilungseigenschaften bereitgestellt werden. Eine Wundkontaktschicht aus den beschriebenen Polyharnstoff/Polyurethan-Copolymeren ist stabil. Jedoch verfügt eine mehrlagiger Wundauflage mit einer derartigen Wundkontaktschicht über eine nur geringe Klebrigkeit am umliegenden Wundgewebe. Die Wundauflage muss bis zur Fixierung der Wundauflage, welche durch einen Verband oder ein Klebepflaster erfolgen kann, mit der Hand an der Wunde gehalten werden.

Bei der modernen Wundbehandlung besteht ein Bedürfnis nach Wundauflagen, welche die Heilung von Wunden beschleunigen und den natürlichen Wundheilungsprozess unterstützen. Geeignete Wundauflagen müssen dabei unterschiedliche erwünschte Eigenschaften in Kombination aufweisen. Neben einer sehr guten Haut- und Gewebeverträglichkeit soll die Wundauflage ein die Wundheilung förderndes feuchtes Milieu gewährleisten, wobei überschüssige Flüssigkeit absorbiert werden sollte. Weiterhin muss die Wundauflage über atraumatische Eigenschaften verfügen, d.h. die Wundauflage muss sich beim Verbandwechsel entfernen lassen, ohne frisch entstandenes Wundgewebe zu beschädigen. Die Wundkontaktschicht spielt dabei sowohl hinsichtlich der erwünschten atraumatischen Eigenschaft und als auch für die Bereitstellung eines die Wundheilung fördernden Milieus aufgrund ihres direkten Kontaktes mit dem Wundgewebe eine herausragende Rolle. Günstig für die Wundheilung ist ein feuchtes Milieu, wobei ein alkalischer pH-Wert, insbesondere ein pH-Wert über 8, vermieden werden sollte. Gleichzeitig soll überschüssige Flüssigkeit aus dem Wundbereich abgeleitet und von der Wundauflage absorbiert werden. Überschüssige Flüssigkeit bzw. Wundexsudat kann ansonsten zur Mazeration des Wundrandes führen. In der Praxis wird von den Anwendern, wie Ärzte oder Pflegepersonal, zudem häufig gewünscht, dass eine Wundauflage bereits vor der Fixierung durch einen Verband oder ein Klebepflaster auf der Wundstelle klebt, d.h. sie soll eine über eine gewisse Oberflächenklebrigkeit (engl. Tack) verfügen. Dies ist insbesondere bei Wundauflagen wünschenswert, welche mit einem weiteren Verbandsmittel, wie einem Fixierverband oder einem Klebepflaster, am Körper fixiert werden. Eine Klebrigkeit der Wundauflage ermöglich der behandelnden Person zunächst eine Wundauflage auf der Wunde locker zu befestigen und danach beispielsweise einen geeigneten Fixierungsverband zurecht zu legen, ohne die Wundauflage gleichzeitig mit einer Hand an die Wunde drücken zu müssen. Eine Wundauflage mit einem Tack ist wesentlich einfacher in der Anwendung, insbesondere wenn der Verband von nur einer Person angelegt wird. Anderseits kann eine zu starke Haftkraft zum einer Verminderung der atraumatischen Eigenschaft der Wundauflage führen und weiterhin beim Verbandwechsel die eine Wunde umgebende unversehrte Haut beschädigen. Ein zu hoher Tack kann auch deshalb weniger wünschenswert sein, weil die Positionierung einer zu stark am Wundgewebe und an der gesunden, die Wunde umgebenden Haut, klebenden Wundauflage nach dem ersten Kontakt mit der Wunde nur noch schlecht korrigiert werden kann.

Ausgehend von den beschriebenen und dem Markt zur Verfügung stehenden Wundauflagen mit einer Wundkontaktschicht aus einer Polyharnstoff/Polyurethan-Copolymer Hydrogelmatrix ist es daher eine Aufgabe der vorliegenden Erfindung, eine verbesserte Wundauflage bereitzustellen. Insbesondere ist es eine Aufgabe der vorliegenden Erfindung eine verbesserte Wundauflage bereitzustellen, die zur Wundheilung in der Granulations- und/ oder Epithelisierungsphase eingesetzt werden kann. Darüber hinaus soll eine atraumatische Wundauflage bereitgestellt werden, die den pathologischen Zustand einer Wunde derart beeinflusst, dass ein normaler, natürlicher Wundheilungsverlauf stattfinden kann. Hierfür soll die Wundauflage eine ausreichende Menge an Feuchtigkeit der Wunde bereitstellen und gleichzeitig einen für die Wunde verträglichen pH-Wert in der Nähe des Neutralpunktes aufweisen. Insbesondere ist es wünschenswert, dass die Wundauflage an der Wundstelle und an der die Wunde umgebenden unversehrten Haut kleben kann, so dass die Wundauflage bis zum Anlegen eines Fixierverbandes oder eines Klebepflasters nicht an der Wundstelle gehalten werden muss. Dabei ist es gleichfalls wünschenswert, dass die Wundauflage ein gutes Absorptionsvermögen aufweist.

Zur Lösung dieser Aufgaben wird eine mehrschichtige Wundauflage gemäß Anspruch 1 vorgeschlagen. Demnach umfasst eine erfindungsgemäße mehrschichtige Wundauflage eine erste Schicht als Wundkontaktschicht, welche eine wasserhaltige Hydrogelmatrix umfasst. Des Weiteren umfasst die Wundauflage mindestens eine zweite absorbierende Schicht. Die Hydrogelmatrix umfasst dabei 37 bis 43 Gew.-% Propylenglykol, ein Vorpolymer mit Isophorondiisocyanatendgruppen (im Folgenden als "Isocyanat" bezeichnet) und ein Diamin auf Polyethylenoxidbasis in einer Menge von insgesamt 12 bis 16,5 Gew.-%, 0 bis 5 Gew.-% eines anorganischen Chlorids, sowie Rest Wasser, wobei das Verhältnis der reaktiven Gruppen von Isocyanat zu den Amingruppen des Diamins 1,25 bis 1,35 betragen soll.

Die vorgeschlagene mehrschichtige Wundauflage beruht auf der überraschenden Entdeckung, dass ein Hydrogel mit einem verbesserter Tack erhalten werden kann, wenn das Hydrogel eine Zusammensetzung umfasst, welche durch eine speziell ausgewählte Menge an Propylenglykol, an einem Vorpolymer mit mit Isophorondiisocyanatendgruppen und an einem Diamin auf Polyethylenoxidbasis gekennzeichnet ist, wobei ein bestimmtes Verhältnis der reaktiven Gruppen von Isocyanat zu den Amingruppen des Diamins vorhanden sein muss.

Eine Wundkontaktschicht mit einer Hydrogelmatrix, welche 37 bis 43 Gew.-% Propylenglykol, ein Vorpolymer mit Isophorondiisocyanatendgruppen und ein Diamin auf Polyethylenoxidbasis in einer Menge von insgesamt 12 bis 16,5 Gew.-%, 0,5 bis 1,5 Gew.-% eines anorganischen Chlorids, sowie Rest Wasser umfasst, wobei das Verhältnis der reaktiven Gruppen von Isocyanat zu den Amingruppen des Diamins 1,25 bis 1,35 beträgt, bleibt auf der Wundestelle haften, d.h. sie weist einen ausreichend starken Tack auf. Zur Bewertung des Tack (Klebrigkeit auf der Wunde und der umgebenden Haut) wurde bei den umfangreichen im Zusammenhang mit der Erfindung durchgeführten Versuchen eine relative Skala von 1 bis 5 herangezogen. Die Bewertung erfolgte anhand des subjektiven Eindrucks einer Person anhand der Klebrigkeit verschiedener Produkte zwischen Zeigefinder und Gel. Auf dem Markt erhältliche Produkte wurden dabei von einer Person in 5 Kategorien eingeteilt. Ein Tack von 1 bedeutet dabei, dass die Wundauflage nicht auf gesunder Haut klebt, während ein Tack von 5 eine sehr hohe Klebrigkeit bedeutet. Wünschenswert ist dabei ein Tack mit dem relativen Wert von 4. Die in der vorliegenden Erfindung offenbarte wasserhaltige Hydrogel-Wundkontaktschicht weist einen ausreichend starken Tack, beispielsweise einen Tack mit einem Wert von 4 auf, wobei die aus dem Stand der Technik bekannten vorteilhaften Eigenschaften der Polyharnstoff/Polyurethan-Copolymer-Hydrogelmatrix erhalten bleiben, oder übertroffen werden.

Die Kombination der wasserhaltigen Hydrogel-Wundkontaktschicht (erste Schicht) und der absorbierenden Schicht (zweite Schicht) weist weiterhin einen für die Wundheilung sehr vorteilhaften pH-Wert in der Nähe des Neutralpunktes, beispielsweise von ca. 7,3 bis 7,5, auf. Das wasserhaltige Hydrogel härtet schnell aus und weist eine zur Verwendung als Wundkontaktschicht in einer mehrschichtigen Wundauflage geeignete Stabilität auf. Eine ausreichende Stabilität der das Hydrogel umfassenden Wundkontaktschicht ist auch dann gegeben, wenn die Wundkontaktschicht Löchern, Kanälen oder Öffnungen aufweist, welche 15 bis 70 % der Fläche der Wundkontaktschicht einnehmen.

Um die erwünschten Eigenschaften der wasserhaltigen Hydrogelmatrix zu erhalten, ist es wesentlich, dass die Zusammensetzung der Hydrogelmatrix einerseits einen Propylenglykolanteil von 37 bis 43 Gew.-% umfasst. Dies ist im Vergleich zu den im Stand der Technik üblicherweise beschriebenen Zusammensetzungen ein wesentlich höherer Gehalt an Propylenglykol. Anderseits soll die Zusammensetzung ein Vorpolymer mit Isophorondiisocyanatendgruppen und ein Diamin auf Polyethylenoxidbasis in einer Menge von insgesamt 12 bis 16,5 Gew.-% umfassen, wobei ein auf den Bereich von 1,25 bis 1,35, eingestelltes Verhältnis ("NCO:NH2") der reaktiven Gruppen von Isocyanat ("NCO") zu den Amingruppen des Diamins ("NH2")vorliegen muss. Weiterhin umfasst die Hydrogelmatrix 0 bis 5 Gew.-% mindestens eines anorganischen Chlorids umfasst. Es wurde gefunden, dass bei einer Abweichung von der in der Erfindung offenbarten Zusammensetzung ein Hydrogel mit vergleichsweise ungünstigeren Eigenschaften erhalten wird, beispielsweise ein Hydrogel mit einem Tack, welches mit einem Wert von weniger als 3 oder mehr als 4 eingestuft wurde, ein Hydrogel mit einem unerwünscht hohem pH-Wert von mehr als 8 oder ein Hydrogel, welches nicht ausreichend aushärtet.

Besonders vorteilhafte Hydrogele können erhalten werden, wenn die Zusammensetzung 37 bis 43 Gew.-% Propylenglykol, vorzugsweise 39 bis 41 Gew.-% Propylenglykol und ein Vorpolymer mit Isophorondiisocyanatendgruppen und ein Diamin auf Polyethylenoxidbasis in einer Menge von insgesamt 12 bis 16,5 Gew.-%, vorzugsweise 14 bis 16 Gew.-%, umfasst, wobei ein auf den Bereich von 1,27 bis 1,33, idealerweise auf 1,29 bis 1,31, eingestelltes Verhältnis der reaktiven Gruppen von Isocyanat zu den Amingruppen des Diamins vorliegt. Derartige Hydrogele weisen hervorragende Eigenschaften hinsichtlich Tack, pH-Wert und Stabilität auf.

Als sehr günstig hat sich dabei erwiesen, wenn die Hydrogelmatrix 0,5 bis 1,5 Gew.-% eines anorganischen Chlorids umfasst, wobei es sich bei dem anorganischen Chlorid vorzugsweise um Natriumchlorid handelt. Insbesondere umfasst die Hydrogelmatrix 0,9 Gew.-% Natriumchlorid.

Das Hydrogel kann erhalten werden, indem Wasser, Propylenglykol und — falls vorhanden - das anorganische Chlorid vermischt werden und anschließend das aufgeschmolzene Amin unter Rühren hinzugegeben wird, bis eine homogene Lösung entstanden ist. Danach wird das Isocyanat zugegeben. Das Gel härtet sehr schnell aus, üblicherweise in wenigen Minuten, beispielsweise in 3 Minuten. Das Aushärten kann, wenn dies erwünscht ist, durch ein Abkühlen der Zusammensetzung nach der Zugabe des Isocyanates verzögert werden. Das Verhältnis der reaktiven Gruppen von Isocyanat zu den Amingruppen des Diamins kann auf übliche Weise anhand der Molekulargewichte der verwendeten Ausgangsstoffe unter Berücksichtigung der Stoffqualität (Reinheit) berechnet und eingestellt werden. Um das Verhältnis der reaktiven Gruppen von Isocyanat zu den Amingruppen des Diamins möglichst genau einzustellen, ist es vorteilhaft, wenn der Amingehalt der Mischung aus Wasser, Propylenglykol, NaCl und Diamin auf eine dem Fachmann bekannte Weise exakt gemessen wird, bevor das Isocyanat zugegeben wird. Das Verhältnis der reaktiven Gruppen von Isocyanat zu den Amingruppen des Diamins kann dann unter Berücksichtigung des gemessenen Amingehaltes anhand der zugegebenen Menge an Isocyanat auf einen Wert von 1,25 bis 1,35, vorzugsweise 1,27 bis 1,33, idealerweise 1,29 bis 1,31, eingestellt werden. Besonders vorteilhafte Gele werden erhalten, wenn das Verhältnis der reaktiven Gruppen von Isocyanat zu den Amingruppen des Diamins auf 1,30 eingestellt wird.

Als besonders geeignet für die erfindungsgemäße mehrschichtige Wundauflage hat sich eine Wundkontaktschicht mit einer Hydrogelmatrix erwiesen, welche folgende Zusammensetzung umfasst:

> 40 Gew.-% Propylenglykol
> 5,7 Gew.-% eines Diamins auf Polyethylenoxidbasis, beispielsweise Jeffamine ED-2003
> 0,9 Gew.-% NaCl
> 9,3 Gew.-% eines Isocyanates, beispielsweise Aquapol PI-13000-3
> Rest Wasser

Bei der Zusammensetzung beträgt das Verhältnis der reaktiven Gruppen von Isocyanat zu den Amingruppen des Diamins 1,30.

Weiterhin weist eine erfindungsgemäße mehrschichtige Wundauflage, welche die Hydrogel-Wundkontaktschicht und eine absorbierende Schicht umfasst, eine unerwartet hohe Absorptionskapazität für Flüssigkeiten auf.

Insbesondere weist eine erfindungsgemäße mehrschichtige Wundauflage, welche die Hydrogel-Wundkontaktschicht und einen hydrophilen Polyurethanschaum mit einem Wasseranteil von mindestens 10 % umfasst, eine besonders hohe Absorptionskapazität für Flüssigkeiten auf.

So kann eine Wundauflage mit einem Hydrogel von 0,8 mm Dicke und einem hydrophilen Polyurethanschaum von 3 mm Dicke (Wassergehalt ca. 58 %) nach 48 Stunden die ca. 8,5-fache Menge, bezogen auf das Eigengewicht, an Wasser absorbieren. Das Hydrogel umfasst 40 Gew.-% Propylenglykol, 9,3 Gew.-% Aquapol (ein Isocyanat), 5,7 Gew.-% Jeffamin (ein Diamin auf Polyethylenoxidbasis), 0,9 Gew.-% NaCl und Rest (ca. 44 Gew.-%) Wasser. Das Verhältnis (NCO:NH2) der reaktiven Gruppen von Isocyanat zu den Amingruppen des Diamins beträgt 1,30. Die vorgenannte Wundauflage zeigt einen die Wundheilung unterstützenden pH-Wert von 7,44 und einen Tack von 4.

Bei der mit mindestens einen zweiten Schicht der mehrschichtigen Wundauflage handelt es sich um eine absorbierende Schicht, welche vorzugsweise einen hydrophilen Polyurethanschaum umfasst, wobei der Polyurethanschaum einen Wasseranteil von mindestens 10 Gew.-% Wasser umfasst.

Besonders bevorzugt umfasst die zweite Schicht dabei einen hydrophilen Polyurethanschaum, der einen Wassergehalt von mindestens 10 Gew.-% Wasser umfasst.

Insbesondere umfasst die zweite Schicht einer erfindungsgemäßen Wundauflage einen hydrophilen Polymerschaum, insbesondere einen hydrophilen Polyurethanschaum, der einen Wasseranteil von mindestens 20 Gew.-% Wasser, insbesondere mindestens 30 Gew.-% und ganz besonders bevorzugt mindestens 35 Gew.-% Wasser umfasst. Hierbei ist weiterhin bevorzugt vorgesehen, dass der hydrophile Polymerschaum, insbesondere der hydrophile Polyurethanschaum einen Wasseranteil von höchstens 80 Gew.-% Wasser, insbesondere höchstens 70 Gew.-% und ganz besonders bevorzugt von höchstens 65 Gew.-% Wasser umfasst. Insbesondere ist dieser Wasseranteil homogen in der Polymermatrix oder der Polyurethanmatrix des Schaums verteilt. Insbesondere umfasst der hydrophile Polymerschaum dabei einen Wasseranteil von mindestens 10 Gew.-% und höchstens 80 Gew.-% Wasser, wobei das Wasser insbesondere homogen in dem hydrophilen Polymerschaum, insbesondere in dem hydrophilen Polyurethanschaum verteilt ist.

Hierbei und im Folgenden soll im Zusammenhang mit der vorliegenden Erfindung — falls nichts anderes angegeben ist — jeder Gehalt eines Inhaltstoffes in Gewichtsprozent (Gew.- %) bezogen auf das Gewicht der den Inhaltsstoff umfassenden Komponente verstanden sein.

Zur Überprüfung der Menge an Wasser in der jeweiligen Komponente soll im Zusammenhang mit der vorliegenden Erfindung die Norm DIN EN 14079 herangezogen werden, wobei die Menge an Wasser wie folgt berechnet wird:

$$W_w = \frac{W_g - W_t}{W_g} \bullet 100\% \qquad (1)$$

mit $W_w$ = Gewicht des Wassers in % bezogen auf das Gesamtgewicht der Komponente $W_g$ = Gewicht der Wasser enthaltenden Komponente

$W_t$ = Gewicht der trockenen Komponente

Damit soll im Zusammenhang mit der vorliegenden Erfindung unter einem hydrophilen Polymerschaum mit einem Wasseranteil von mindestens 10 Gew.-% oder einem hydrophilen Polyurethanschaum mit einem Wasseranteil von mindestens 10 Gew.-% ein solcher Polymerschaum oder Polyurethanschaum verstanden sein, der mindestens 10 Gew.-% Wasser umfasst, wobei das Wasser von dem Polymerschaum oder dem Polyurethanschaum freigesetzt werden kann. Im Unterschied hierzu ist nicht der Anteil an Wasser zu verstehen, der eventuell zur Bildung beispielsweise bei der Polymerisation der Ausgangsprodukte des Polymerschaums oder des Polyurethanschaums verwendet wird. Dieses Wasser wird kovalent gebunden und steht nicht der Wundbehandlung zur Verfügung. Darüber hinaus soll auch nicht ein Wasseranteil verstanden sein, der produktionsbedingt bei der Herstellung des Schaums verwendet wird. Dieser Wasseranteil wird nach oder während der Bildung des Polymerschaums dem Schaum meist durch Trocknen, beispielsweise durch Trocknen in einem Ofen, entzogen und steht somit auch nicht der Wundbehandlung zur Verfügung. Damit weist eine erfindungsgemäße Wundauflage einen Polymerschaum oder einen Polyurethanschaum auf, der einen Wasseranteil umfasst, der deutlich über einem eventuell durch die Herstellung nach Trocknung bedingten Restgehalt an Wasser übersteigt.

Weiterhin bevorzugt umfasst eine erfindungsgemäße Wundauflage einen hydrophilen Polyurethanschaum, der einen Retentionswert R von mindestens 20% aufweist. Hierbei ist weiterhin bevorzugt vorgesehen, dass der hydrophile Polyurethanschaum einen Retentionswert R von mindestens 30 %, insbesondere von mindestens 40 %, insbesondere mindestens 40 % und ganz besonders bevorzugt von mindestens 50 % aufweist. Unabhängig hiervon kann weiterhin bevorzugt vorgesehen sein, dass die Wundauflage einen hydrophilen Polyurethanschaum aufweist, der einen Retentionswert R von höchstens 90 %, insbesondere von höchstens 80 % und ganz besonders von höchstens 70 % aufweist. Der Retentionswert R wird dabei gemäß einer hierin beschriebenen Methode bestimmt.

Ganz besonders bevorzugt umfasst eine erfindungsgemäße Wundauflage einen hydrophilen Polyurethanschaum, der einen Wasseranteil von mindestens 10 Gew.-% umfasst, wobei der Wasseranteil dem Retentionswert R des Polyurethanschaums entspricht.

Somit kann eine Wundauflage bereitgestellt werden, die im Vergleich zu bekannten Hydrogel-Wundauflagen eine höhere Absorptionskapazität und gleichzeitig einen hohen Wassergehalt aufweist. Dieses Wasser kann die Wundauflage bei der Behandlung von Wunden abgeben und gleichzeitig eventuell vorhandenes Wundexsudat aufnehmen. Im Vergleich zu bekannten Polymerschaum-Wundauflagen kann eine Wundauflage bereitgestellt werden, die die Wundoberfläche ausreichend mit Feuchtigkeit oder Wasser versorgt und gleichzeitig eine ausreichende Absorptionskapazität aufweist. Hierdurch werden die Wundheilung eventuell negativ beeinflussende Faktoren der Wundoberfläche entzogen und gleichzeitig Feuchtigkeit und Wasser in einer ausreichenden Menge zur Verfügung gestellt. Aufgrund der geringeren Absorptionskapazität gegenüber trockenen Polymerschäumen, die kein freisetzbares Wasser umfassen und meist sehr schnell sehr viel Wundsekret aufnehmen, wodurch eine trockene Wundoberfläche gebildet wird, ist diese Wundauflage hervorragend geeignet in der Epithelisierungs- oder Granulationsphase der Wundheilung eingesetzt zu werden.

Insbesondere ergibt sich bei der erfindungsgemäßen mehrschichtigen Wundauflage ein Vorteil durch die Kombination eines absorbierenden, die Wunde feucht haltenden hydrophilen Polymerschaums mit einer Hydrogel-Wundkontaktschicht, welche einen die Wundheilung nicht beeinträchtigenden pH-Wert, beispielsweise zwischen 7,3 und 7,5, aufweist.

Als hydrophiler Polymerschaum kann im Zusammenhang mit der vorliegenden Erfindung jeder heute in der modernen Wundbehandlung übliche hydrophile Polymerschaum verwendet werden, der einen Wasseranteil in sein Polymergerüst aufnimmt und dennoch eine ausreichende Absorption aufweist. Damit ist im Zusammenhang mit der vorliegenden Erfindung unter einem hydrophilen Polymerschaum ein solcher Polymerschaum zu verstehen, der Flüssigkeiten absorbieren und speichern kann und zumindest einen Teil dieser Flüssigkeiten wieder abgeben kann. Insbesondere können als Polymerschaum ein hydrophiler Polyurethanschaum, ein hydrophiler Polyetherschaum, ein hydrophiler Polyurethan-Polyether-Copolymerschaum, ein hydrophiler Polyvinylacetatschaum, hydrophiler Polyvinylalkoholschaum, ein hydrophiler Kollagenschaum, ein hydrophiler Chitosanschaum oder Mischungen dieser Schäume eingesetzt werden. Ganz besonders bevorzugt kann als Polymerschaum ein hydrophiler Polyurethanschaum eingesetzt werden.

Erfindungsgemäß sollen solche Polymerschäume, insbesondere Polyurethanschäume eingesetzt werden, die eine hohe Absorptionskapazität aufweisen. Diese Absorptionskapazität soll vorhanden sein, obwohl der Polymerschaum, insbesondere der Polyurethanschaum einen Anteil seines Eigengewichtes an Wasser in seine Polymermatrix bzw. seine Polyurethanmatrix aufgenommenen hat. Gemäß einer Weiterbildung der Erfindung umfasst damit eine erfindungsgemäße Wundauflage einen hydrophilen Polymerschaum, insbesondere einen hydrophilen Polyurethanschaum, der einen Wasseranteil von mindestens 10 Gew.-% und höchstens 80 Gew.-% Wasser umfasst und der eine freie Absorption $A_2$ von mindestens 10 g/ g, insbesondere mindestens 12 g/ g und ganz besonders bevorzugt von mindestens 15 g/ g aufweist, wobei die freie Absorption $A_2$ gemäß der DIN-EN 13726-1 (2002) bestimmt wird. Hierbei ist die freie Absorption $A_2$ die freie Absorption des Wasser enthaltenden Polymerschaums oder des Wasser enthaltenden Polyurethanschaums.

Weiterhin bevorzugt umfasst eine erfindungsgemäße Wundauflage einen hydrophilen Polymerschaum, insbesondere einen hydrophilen Polyurethanschaum, der einen Wasseranteil von mindestens 10 Gew.-% und höchstens 80 Gew.-% Wasser umfasst und der eine freie Absorption $A_1$ von mindestens 10 g/ g, insbesondere mindestens 12 g/ g und ganz besonders bevorzugt von mindestens 15 g/ g aufweist, wobei die freie Absorption $A_1$ gemäß der DIN-EN 13726-1 (2002) bestimmt wird. Hierbei ist die freie Absorption $A_1$ die freie Absorption des trockenen Polymerschaums oder des trockenen Polyurethanschaums.

Gemäß einer Weiterbildung der vorliegenden Erfindung umfasst eine erfindungsgemäße Wundauflage damit auch eine zweite Schicht mit einer ersten und einer zweiten Seite als absorbierende Schicht, wobei die zweite Schicht den hydrophilen Polymerschaum oder den hydrophilen Polyurethanschaum umfasst. Insbesondere weist dabei die erste Schicht einen direkten Kontakt zu der zweiten Schicht auf. Es kann jedoch auch vorgesehen sein, dass dabei die zweite Schicht als absorbierende Schicht einen direkten Kontakt zur einer Wundkontaktschicht aufweist. Hierbei weist eine Wundkontaktschicht einen direkten Kontakt zur Wunde auf.

Falls als hydrophiler Polymerschaum ein hydrophiler Polyurethanschaum verwendet wird, kann eine Wundauflage bereitgestellt werden, die gegenüber Wundauflagen mit trockenen hydrophilen Polyurethanschäumen eine sehr viel geringere Scherbelastung auf die Wunde ausübt. Durch einen Wasseranteil von mindestens 10 Gew-% Wasser in dem Polymerschaum kann eine Wundauflage mit einem vorkonditionierter hydrophilen Polyurethanschaum bereitgestellt werden, die bei annähernd gleicher Absorption von Flüssigkeiten gegenüber einer Wundauflage mit einem trockenen hydrophilen Polyurethanschaum ein sehr viel geringeres Quellvermögen aufweist. Durch das geringere Quellvermögen des vorkonditionierten Polyurethanschaums herrschen somit innerhalb der Wundauflage geringere Scherkräfte in Bezug auf weitere Lagen oder Materialien oder in Bezug auf eine zu behandelnde Wunde. Hierdurch kann eine Polyurethanschaum-Wundauflage bereitgestellt werden, die besonders wundheilungsfördernd ist.

Damit ist gemäß einem weiterführenden Gedanken auch eine Wundauflage Gegenstand der Erfindung, die eine zweite Lage mit einer ersten Seite und einer zweiten Seite umfasst, wobei die Lage einen hydrophilen Polyurethanschaum umfasst, der einen Wasseranteil von mindestens 10 Gew.-% umfasst und der ein Quellvermögen $\Delta V_1$ von höchstens 80 % aufweist. Insbesondere weist der hydrophile Polyurethanschaum dabei ein Quellvermögen $\Delta V_1$ von höchstens 60 %, insbesondere von höchstens 40 %, insbesondere von höchstens 30 % und ganz besonders bevorzugt von höchstens 20 % auf. Dabei kann weiterhin vorteilhaft vorgesehen sein, dass der Polyurethanschaum ein Restquellvermögen $\Delta V_1$ von mindestens 5 % aufweist. Dieses Restquellvermögen kann ausgenutzt werden, damit die Wundauflage während der Absorption einen besseren Kontakt zum Wundgrund annehmen kann.

Hierbei soll unter dem Quellvermögen $\Delta V_1$ eines Polyurethanschaums die Volumenzunahme verstanden sein, die ein Polyurethanschaum, der seine Absorptionskapazität vollständig erschöpft hat, im Vergleich zu einem Polymerschaum mit einem Wassergehalt von mindestens 10 Gew.-% Wasser erfährt. Das Quellvermögen soll dabei gemäß einem hierin beschrieben Test ermittelt werden.

Als Polyurethanschaum kann im Zusammenhang mit der vorliegenden Erfindung jeder heute in der modernen Wundbehandlung übliche hydrophile Polyurethanschaum verwendet werden, der einen Wasseranteil in seine Polyurethanmatrix aufnimmt und eine ausreichende Absorption aufweist. Damit ist im Zusammenhang mit der vorliegenden Erfindung unter einem hydrophilen Polyurethanschaum ein solcher Polyurethanschaum zu verstehen, der eine Flüssigkeit in seine Polyurethanmatrix und in seine Poren aufnehmen und speichern, somit absorbieren kann, und zumindest einen Teil der aufgenommene Flüssigkeit wieder abgeben kann. Als hydrophile Polymerschäume sind hierbei insbesondere offenporige, hydrophile Polyurethanschäume geeignet. Dem gemäß umfasst eine besonders bevorzugte Wundauflage eine zweite Schicht, die einen offenporigen, hydrophilen Polyurethanschaum umfasst.

Gemäß einer bevorzugten Ausbildung der Erfindung umfasst die erfindungsgemäße Wundauflage weiterhin einen hydrophilen Polyurethanschaum, wobei der Polyurethanschaum im trockenen Zustand eine Bruchdehnung nach ISO1798-M1 von 50 bis 105 kPa, vorzugsweise von 72 bis 94 kPA aufweist. Ein hydrophiler Polyurethanschaum, welcher im trockenen Zustand eine Bruchdehnung nach ISO1798-M1 von 50 von 72, insbesondere 72 bis 94 kPA aufweist, ist für die erfindungsgemäße Wundauflage besonders geeignet, da eine Wundauflage mit einem solchen Polyurethanschaum über eine ausreichende Stabilität verfügt und sich dabei gleichzeitig dem Untergrund ausreichend gut anschmiegt.

Weiterhin bevorzugt umfasst eine erfindungsgemäße Wundauflage einen hydrophilen Polymerschaum, insbesondere einen hydrophilen Polyurethanschaum, der eine durchschnittliche Porengröße von weniger als 1000 $\mu$m, insbesondere 100 bis 1000 $\mu$m, insbesondere 100 bis 500 $\mu$m und ganz besonders bevorzugt 100 bis 300 $\mu$m aufweist. Im Zusammenhang mit der erfindungsgemäßen mehrschichtigen Wundeauflage hat sich insbesondere ein hydrophiler Polyurethanschaum mit einer Porengröße von 150 bis 220 $\mu$m, wobei die Porengröße mikroskopisch bestimmt wurde, als besonders vorteilhaft erwiesen. Hierbei kann insbesondere vorgesehen sein, dass die durchschnittliche Porengröße an der ersten Seite der zweiten Schicht gleich groß ist wie die Porengröße im inneren der zweiten Schicht und/ oder gleich groß ist wie an der zweiten Seite der zweiten Schicht. Weiterhin bevorzugte hydrophile Polyurethanschäume weisen eine Dichte von weniger als 150 kg/ m$^3$, insbesondere weniger als 140 kg/ m$^3$ und ganz besonders bevorzugt 50 bis 120 kg/ m$^3$ auf. Als besonders vorteilhafte Ausführungen haben sich weiterhin Wundauflagen gezeigt, die einen hydrophilen Polymerschaum, insbesondere einen Polyurethanschaum, umfassen, dessen Schichtdicke 0,1 bis 10,0 mm aufweist. Insbesondere weist damit eine erfindungsgemäße Wundauflage eine absorbierende Schicht mit einer Schichtdicke von 0,1 bis 6,0 mm, insbesondere von 0,5 bis 6,0 mm und ganz besonders bevorzugt von 2,0 bis 5,5 mm auf. Wundauflagen mit solchen Schichtdicken zeigen einerseits die Fähigkeit ein von einer Wunde abgegebenes Wundexsudat aufzunehmen zu können und gleichzeitig eine ausreichende Menge an Wasser oder Feuchtigkeit einer Wunde bereitstellen zu können. Dieser vorteilhafte Effekt wird durch Wundkontaktschicht, die eine wasserhaltige Hydrogelmatrix mit einer Schichtdicke von 0,6 bis 1,0 mm umfasst, weiter verstärkt. Gemäß einer bevorzugten Ausführungsform der Erfindung umfasst die mehrschichtige Wundauflage a) eine erste Schicht als Wundkontaktschicht, die eine wasserhaltige Hydrogelmatrix mit einer Schichtdicke von 0,8 mm umfasst, und b) eine zweite absorbierende Schicht, die einen hydrophilen Polyurethanschaum mit einer Schichtdicke von 4,0 mm umfasst, auf, wobei die erste Schicht und die zweite Schicht in direktem Kontakt zueinander stehen.

Gemäß einer weiteren sehr vorteilhaften Ausbildung der Erfindung umfasst die mehrschichtige Wundauflage a) eine erste Schicht als Wundkontaktschicht, die eine wasserhaltige Hydrogelmatrix mit einer Schichtdicke von 0,8 mm umfasst, und b) eine zweite absorbierende Schicht, die einen hydrophilen Polyurethanschaum mit einer Schichtdicke von 2,8 mm umfasst, auf, wobei die erste Schicht und die zweite Schicht in direktem Kontakt zueinander stehen. Diese Schichtdicken können an jeder Stelle der Wundkontaktschicht gleich sein oder in verschiedenen Bereichen der Wundkontaktschicht verschiedene Werte annehmen. Insbesondere ist auch vorgesehen, dass die absorbierende Schicht oder der Polyurethanschaum abgeflachte Ränder aufweist.

Im Zusammenhang mit der vorliegenden Erfindung ist weiterhin vorgesehen, dass die erfindungsgemäße Wundauflage als Wundkontaktschicht eine wasserhaltige Hydrogelmatrix umfasst, die mindestens 39,5 Gew.-%, insbesondere mindestens 43 Gew.-%, Wasser umfasst, wobei die Hydrogelmatrix weiterhin bevorzugt höchstens 50 Gew.-% Wasser umfasst. Somit kann eine Wundauflage bereitgestellt werden, die eine für natürliche Wundheilung ausrechende Menge Feuchtigkeit zur Verfügung stellt.

Als wasserhaltige Hydrogelmatrices können im Zusammenhang mit der vorliegenden Erfindung insbesondere Hydrogelmatrices verwendet werden, die eine zusammenhängende, diskrete Schicht bilden und unter Druck kein Wasser abgeben. Im Zusammenhang mit der vorliegenden Erfindung handelt es sich dabei um Hydrogelmatrices, die ein Polyurethan-Polyharnstoff-Copolymer umfassen. Diese Hydrogelmatrices sind besonders gut geeignet Wasser einzulagern und dieses Wasser an eine Wunde abzugeben.

Weiterhin soll die wasserhaltige Hydrogelmatrix 37 bis 43 Gew.-% Propylenglykol enthalten. Dieser Alkohol ist hervorragend als Feuchtigkeitsspender geeignet und stellt somit für die die Wunde umgebende Haut eine pflegende Komponente dar.

Dabei soll die wasserhaltige Hydrogelmatrix insbesondere 37 bis 43 Gew.-% des mehrwertigen Alkohols Propylenglykol umfassen. Insbesondere umfasst die Hydrogelmatrix 39 bis 41 Gew.-% Propylenglykol und ganz besonders bevorzugt 40 Gew.-% Propylenglykol

Weiterhin ist es für die Erfindung wesentlich, dass das Verhältnis (NCO:NH2) der reaktiven Gruppen von Isocyanat (NCO) zu den Amingruppen des Diamins (NH2) 1,25 bis 1,35 beträgt, da nur dann der vorteilhafte Tack der Hydrogelmatrix erhalten wird, ohne andere Eigenschaften, wie zum Beispiels den pH-Wert oder die Stabilität des Hydrogels in unerwünschter Weise zu verändern. Insbesondere soll das Verhältnis der reaktiven Gruppen von Isocyanat zu den Amingruppen des Diamins 1,27 bis 1,33, vorzugsweise 1,29 bis 1,31 betragen. Besonders vorteilhaft ist ein Verhältnis der reaktiven Gruppen von Isocyanat zu den Amingruppen des Diamins im Bereich 1,30.

Weiterhin ist vorgesehen, dass die wasserhaltige Hydrogelmatrix mindestens ein anorganisches Chlorid umfasst. Besonders geeignet sind in diesem Zusammenhang Natriumchlorid, Kaliumchlorid, Magnesiumchlorid, Kalziumchlorid oder Mischungen hiervon. Diese Salze simulieren in besonders guter Weise das Elektrolyt-Gemisch in einem von einer Wunde abgegebenen Wundserum. Damit stellt eine diese Salze umfassende Hydrogelmatrix durch ähnliche osmotische Verhältnisse zwischen Hydrogel und Wundexsudat einer Wunde ein besonders wundheilungsförderndes Klima zur Verfügung.

Hierbei kann vorgesehen sein, dass die Hydrogelmatrix 0 bis 5 Gew.-% mindestens eines anorganischen Chlorids umfasst. Insbesondere umfasst die Hydrogelmatrix 0,1 bis 3 Gew.-% eines anorganischen Chlorids und ganz besonders bevorzugt 0,5 bis 1,5 Gew.-% eines anorganischen Chlorids. Als sehr günstig hat sich dabei eine Hydrogelmatrix erwiesen, welche 0,9 Gew.-% Natriumchlorid enthält.

Eine wasserhaltige Hydrogelmatrix umfassend 37 bis 43 Gew.-% Propylenglycol, ein Vorpolymer mit Isophorondiisocyanatendgruppen und ein Diamin auf Polyethylenoxidbasis in einer Menge von insgesamt 12 bis 16,5 Gew.-%, 0,5 bis 1,5 Gew.-% eines anorganischen Chlorids und Rest Wasser, wobei das Verhältnis der reaktiven Gruppen von Isocyanat zu den Amingruppen des Diamins 1,25 bis 1,35 beträgt, weist eine freie Absorption $A_3$ (gemessen nach DIN EN 13726-1 (2002)) von mindestens 1 g/ g und höchstens 10 g/ g auf. Die wasserhaltige Hydrogelmatrix stellt ein nicht reizendes, flüssigkeitsabsorbierendes, vor Bakterien schützendes, polsterndes, hautartiges Medium bereit und ist somit besonders gut als Wundkontaktschicht geeignet.

Gemäß der vorliegenden Erfindung ist die erste Schicht eine Wundkontaktschicht. Als Wundkontaktschicht ist hierbei eine Schicht zu verstehen, die einen direkten Kontakt zur Wunde aufweisen kann. Die Wundkontaktschicht kann gemäß der vorliegenden Erfindung einzig und allein dazu dienen, dass der Polyurethanschaum von der zu behandelnden Wunde beabstandet wird. Die Wundkontaktschicht kann auch weitere Funktionen in Bezug auf die Wundauflage als auch auf die zu behandelnde Wunde ausüben. Insbesondere kann eine erfindungsgemäße Wundauflage eine Wundkontaktschicht umfassen, wobei die Wundkontaktschicht neben der Hydrogelmatrix, weiterhin einen Polymerfilm, eine Hydrokolloidmatrix, ein Polymernetz, ein Nonwoven oder ein Adhäsiv umfasst.

Als besonders vorteilhafte Ausführungen haben sich weiterhin Wundauflagen gezeigt, die eine Hydrogelmatrix umfassen, deren Schichtdicke 0,1 bis 5,0 mm aufweist. Insbesondere weist damit eine erfindungsgemäße Wundauflage eine Hydrogelmatrix mit einer Schichtdicke von 0,1 bis 5,0 mm, insbesondere von 0,5 bis 5,0 mm und ganz besonders bevorzugt von 0,5 bis 3,0 mm auf. Wundauflagen mit solchen Schichtdicken als Wundkontaktschicht zeigen einerseits keine Wundverklebung und andererseits die Fähigkeit ein von einer Wunde abgegebenes Wundexsudat aufzunehmen und an eine absorbierende Schicht weiterzuleiten. Eine hinsichtlich ihres Zusammenwirkens mit der zweiten absorbierenden Schicht besonders geeignete Wundkontaktschicht umfasst eine Hydrogelmatrix mit einer Schichtdicke von 0,6 bis 1,0 mm, insbesondere 0,8 mm. Diese Schichtdicken können an jeder Stelle der Wundkontaktschicht gleich sein oder in verschiedenen Bereichen der Wundkontaktschicht verschiedene Werte annehmen.

Weiterhin bevorzugt kann die Hydrogelmatrix Kanäle umfassen, insbesondere konische Kanäle, zum Durchtritt von Flüssigkeiten von der ersten zur zweiten Seite. Hierdurch kann insbesondere ein verbesserter Durchtritt für Wundexsudat bereitgestellt werden. Hierbei ist besonders bevorzugt vorgesehen, dass die Kanäle einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sind. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Dabei ist ganz besonders bevorzugt vorgesehen, dass die erste Seite Öffnungen aufweist, die im Vergleich zu den auf der zweiten Seite befindlichen Öffnungen größer sind.

Gemäß einer Weiterbildung der Erfindung kann auch vorgesehen sein, dass die Hydrogelmatrix Öffnungen aufweist, die einen Durchmesser von 0,5 bis 10 mm aufweisen. Insbesondere weist die Wundkontaktschicht oder die Hydrogelmatrix Öffnungen auf, die einen Durchmesser von 1 bis 8 mm aufweisen. Ganz besonders bevorzugt weist die Wundkontaktschicht oder die Hydrogelmatrix auf der wundzugewandten ersten Seite Öffnungen auf, die einen Durchmesser von 2 bis 6 mm aufweisen, wobei die zweite Seite der Wundkontaktschicht oder der Hydrogelmatrix in direktem Kontakt mit dem Polyurethanschaum steht.

Es kann jedoch auch vorgesehen sein, dass zwischen der ersten Schicht und der zweiten Schicht, insbesondere zwischen der ersten Schicht als Wundkontaktschicht und der zweiten Schicht als absorbierender Schicht, eine Übergangsschicht angeordnet ist. In dieser Ausführungsform weist eine erfindungsgemäße Wundauflage zwischen der Hydrogelmatrix und dem Polymerschaum, insbesondere zwischen der Hydrogelmatrix und dem Polyurethanschaum eine Schicht auf, die beide Materialen umfasst. Diese Übergangsschicht kann ebenso wie die Wundkontaktschicht Kanäle, Öffnungen oder Löcher aufweisen. Falls die Übergangsschicht Kanäle, öffnungen oder Löcher aufweist, sind gemäß einer weiter bevorzugten Ausführungsform diese Kanäle, Öffnungen oder Löcher mit Polyurethanschaum ausgefüllt. Weiterhin bevorzugt sind diese Kanäle, Öffnungen oder Löcher kongruent zu den Kanälen, Öffnungen oder Löchern der Wundkontaktschicht. Durch die Anordnung einer solchen Übergangangsschicht kann eine Wundauflage bereitgestellt werden, die ein Laminat aus einem Polyurethanschaum und einer Hydrogelmatrix umfasst, das einen besonders festen Zusammenhalt zwischen der absorbierenden Schicht und der Wundkontaktschicht aufweist.

Als Wundkontaktschicht findet gemäß der vorliegenden Erfindung ein Material Verwendung finden, das keinen negativen Einfluss auf die Wundheilung ausübt. Diese Wundkontaktschicht kann einzig und allein dazu dienen, dass der Polyurethanschaum oder die Hydrogelmatrix von der zu behandelnden Wunde beabstandet wird, als auch dass diese Wundkontaktschicht weitere Funktionen in Bezug auf die Wundauflage als auch auf die zu behandelnde Wunde ausübt. Insbesondere kann eine erfindungsgemäße Wundauflage eine Wundkontaktschicht mit einer ersten Seite und einer zweiten Seite umfassen, wobei die Wundkontaktschicht eine Hydrogelmatrix umfasst.

Gemäß einer Weiterbildung der erfindungsgemäßen Wundauflage kann vorgesehen sein, dass die Wundkontaktschicht eine Vielzahl an Kanälen, Öffnungen oder Löcher zum Durchtritt von Flüssigkeiten umfasst. Insbesondere ist dabei vorgesehen, das die Wundkontaktschicht Kanäle aufweist, die einen Durchtritt von Wundsekret von der ersten Seite zur zweiten Seite bilden. In dieser Ausführungsform ist vorgesehen, dass die erste Seite der Wundkontaktschicht einen direkten Kontakt zu einer zu behandelnden Wunde und die zweite Seite der Wundkontaktschicht einen direkten Kontakt mit der ersten Seite der absorbierenden Schicht aufweist.

Insbesondere kann auch vorgesehen sein, dass die Wundkontaktschicht Kanäle, Öffnungen oder Löcher aufweist, die einen Durchmesser von 0,5 bis 10 mm aufweisen. Insbesondere weist die Wundkontaktschicht Kanäle, Öffnungen oder Löcher auf, die einen Durchmesser von 3 bis 6 mm aufweisen. Ganz besonders bevorzugt weist die Wundkontaktschicht auf der im anwendungsgerechten Zustand der Wundauflage der Wunde zugewandten, ersten Seite Öffnungen auf, die einen Durchmesser von 4 bis 5 mm aufweisen, wobei die zweite Seite der Wundkontaktschicht in direktem Kontakt mit dem Polyurethanschaum steht. Hierbei und im Folgenden bezieht sich der Durchmesser der Kanäle, Öffnungen oder Löcher auf den mittleren Durchmesser, falls es sich bei den Kanälen, Öffnungen oder Löchern um konisch gestaltete Kanäle, Öffnungen oder Löcher handelt. Unter mittlerem Durchmesser wird in diesem Zusammenhang ein Durchmesser verstanden, welcher in der Mitte der betreffenden Schicht, beispielsweise der Wundkontaktschicht, vorhanden ist. Dieser mittlere Durchmesser kann bei konisch geformten Kanälen, Öffnungen oder Löchern kleiner sein, als der Durchmesser der Kanälen, Öffnungen oder Löchern auf ersten Seite und gleichzeitig größer als Durchmesser Kanälen, Öffnungen oder Löchern der zweiten Seite. In analoger Weise ist unter einem Abstand der Kanälen, Öffnungen oder Löchern der mittlere Abstand der Kanälen, Öffnungen oder Löchern gemeint. Entsprechend wird unter dem Abstand der Reihen zueinander der mittlere Abstand der Reihen zueinander verstanden.

Weiterhin bevorzugt kann die Wundkontaktschicht eine Vielzahl an Kanälen, Öffnungen oder Löcher zum Durchtritt von Flüssigkeiten aufweisen, wobei die Kanäle, Öffnungen oder Löcher an der ersten Seite der Wundkontaktschicht eine Fläche von höchstens 70 % der Fläche der ersten Seite der Wundkontaktschicht einnehmen. Hierbei ist weiterhin bevorzugt vorgesehen, dass die Kanäle, Öffnungen oder Löcher eine Fläche von höchstens 70 %, insbesondere höchstens 60 %, und ganz besonders bevorzugt von höchstens 50 % der Fläche der ersten Seite der Wundkontaktschicht einnehmen. Ganz besonders bevorzugt weist die Wundkontaktschicht Kanäle, Öffnungen oder Löcher auf, die an der ersten Seite der Schicht eine Fläche von mindestens 15 % der Fläche der ersten Seite der Wundkontaktschicht einnehmen. Ganz besonders bevorzugt weist die Wundkontaktschicht weiterhin Kanäle, Öffnungen oder Löcher auf, die an der ersten Seite der Schicht eine Fläche von mindestens 40 % und höchstens 47 % der Fläche der ersten Seite der Wundkontaktschicht einnehmen. Ganz besonders weist die Wundauflage.

Die Anordnung der Kanäle, Öffnungen oder Löcher kann nach einer sehr vorteilhaften Ausführungsform in versetzten Reihen ausgeführt sein. Dabei hat sich insbesondere ein mittlerer Durchmesser der Kanäle, Öffnungen oder Löcher von 4,5 mm als vorteilhaft erwiesen. Der Abstand der Mittelpunkte der Kanäle, Öffnungen oder Löcher einer Reihe beträgt dabei 6,5 mm, so dass zwischen den Kanälen, Öffnungen oder Löchern ein Gelsteg mit einer Breite von 2 mm entsteht. Der Abstand der Reihen zueinander ist mit 5,63 mm so gewählt, dass zwischen den Mittelpunkten von drei Löchern ein gleichseitiges Dreieck entsteht. Somit ist eine homogene Verteilung der offenen Fläche von 43,5% über die Produktfläche gegeben. Ist der Durchmesser der Löcher wesentlich kleiner, kann insbesondere dickflüssiges Exsudat die Gelschicht weniger gut passieren. Wesentlich größere Löcher führen dagegen entweder zu einer kleineren Gelfläche oder zu sehr breiten Stegen, welche wiederum an dieser Stelle das Wundexsudat stauen können. Eine kleinere Gelfläche führt dazu, dass das Gesamtprodukt weniger Haftfläche besitzt und somit der Tack vermindert werden kann.

Gemäß einem weiteren weiterführenden Gedanken der vorliegenden Erfindung, ist auch eine Wundauflage Gegenstand der vorliegenden Erfindung, die zwischen der Hydrogelmatrix und dem hydrophilen Polymerschaum eine Barriereschicht aufweist. Eine solche Barriereschicht kann beispielsweise einen Polymerfilm umfassen, der mit Öffnungen versehen ist.

Weiterhin kann eine erfindungsgemäße Wundauflage eine Trägerschicht umfassen. Diese Trägerschicht kann aus verschiedenen Materialien bestehen. Üblicherweise werden in Wundauflagen textile Trägermaterialien, Nonwoven, Polymerfilme oder Polymerschäume eingesetzt. Diese Trägerschicht kann direkten Kontakt oder indirekten Kontakt zu der zweiten Seite der absorbierenden Schicht oder dem hydrophilen Polymerschaums aufweisen. Bei einem direkten Kontakt wird die Trägerschicht direkt auf die absorbierende Lage oder den Polyurethanschaum auflaminiert, wogegen bei einem indirekten Kontakt die Trägerschicht mittels eines Adhäsivs auf die absorbierende Schicht oder dem Polyurethanschaum aufgebracht ist. Dabei kann das Adhäsiv vollflächig oder lediglich in Teilbereichen zwischen der Trägerschicht und der absorbierenden Schicht aufgebracht sein.

Als Trägerschicht einer erfindungsgemäßen Wundauflage können insbesondere Polymerfilme oder Polymerschäume eingesetzt werden. Ganz besonders bevorzugt sind Polymerfilme oder Polymerschäume, die wasserundurchlässig sind und die eine hohe Wasserdampfdurchlässigkeit aufweisen. Hierzu sind besonders Filme oder Schäume geeignet, die aus Polyurethan, Polyetherurethan, Polyesterurethan, Polyether-Polyamid-Copolymeren, Polyacrylat oder Polymethacrylat gefertigt werden. Insbesondere ist als Trägerschicht ein wasserundurchlässigen und wasserdampfdurchlässigen Polyurethanfilm oder einen wasserundurchlässigen und wasserdampfdurchlässigen Polyurethanschaum geeignet. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 $\mu$m, insbesondere 20 bis 40 $\mu$m und ganz besonders bevorzugt von 25 bis 30 $\mu$m aufweisen. Die Wasserdampfdurchlässigkeit des Polymerfilms der Wundauflage weist vorzugsweise mindestens 750 g/ m$^2$/ 24 Std., insbesondere mindestens 1000 g/ m2/ 24 Std. und ganz besonders bevorzugt mindestens 2000 g/ m$^2$/ 24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m$^2$ zusammen mit dem Film eine Wasserdampfdurchlässigkeit von mindestens 800 g/ m$^2$/ 24 Std. und vorzugsweise von mindestens 1000 g/ m$^2$/ 24 Std. (gemessen nach DIN EN 13726) aufweisen.

Gemäß einem weiterführenden Gedanken der vorliegenden Erfindung, ist auch eine mehrschichtige Wundauflage umfassend eine erste Schicht als Wundkontaktschicht, die eine wasserhaltige Hydrogelmatrix umfasst, eine zweite Schicht als absorbierende Schicht, die einen hydrophilen Polymerschaum umfasst, eine Trägerschicht und eine Verteilerschicht, ebenfalls Gegenstand der vorliegenden Erfindung. Insbesondere ist die absorbierende Schicht mit der Wundkontaktschicht verbunden. Eine solche Wundauflage weist besonders vorteilhaft eine Verteilerschicht zwischen der Trägerschicht und der absorbierenden Schicht auf, die aus einem hydrophilen Polyurethanschaum besteht. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt.

Gemäß einem weiteren weiterführenden Gedanken der vorliegenden Erfindung, ist auch eine mehrschichtige Wundauflage umfassend eine erste Schicht, die eine wasserhaltige Hydrogelmatrix als Wundkontaktschicht umfasst, eine zweite Schicht als absorbierende Schicht, die einen hydrophilen Polymerschaum umfasst, eine dritte Schicht als Verteilerschicht und eine Trägerschicht, Gegenstand der vorliegenden Erfindung. Hierbei können als Trägerschicht alle oben erwähnten Materialien eingesetzt werden.

In den Zeichnungen zeigen:

Figur 1:      Eine erste erfindungsgemäße Wundauflage (Sicht auf die Wundkontaktschicht)
Figur 2:      Eine zweite erfindungsgemäße Wundauflage im Querschnitt
Figur 2a:    Ein Teilausschnitt der zweiten erfindungsgemäße Wundauflage im Querschnitt
Figur 3      Eine dritte erfindungsgemäße Wundauflage im Querschnitt
Figur 3a    Ein Teilausschnitt der dritten erfindungsgemäße Wundauflage im Querschnitt

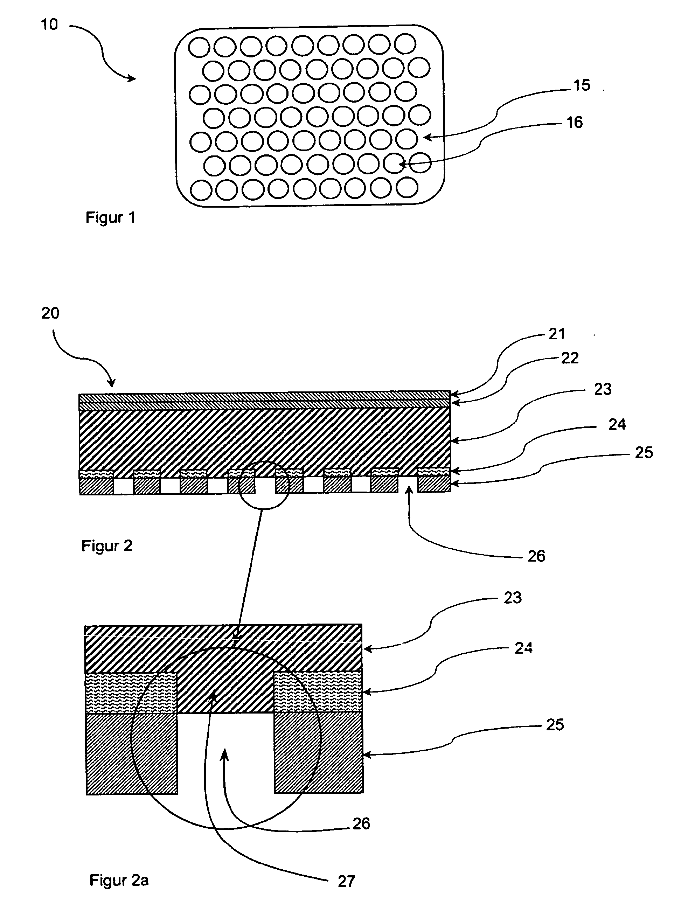

Mit Figur 1 ist eine erste mehrschichtige Wundauflage (10) mit Sicht auf die Wundkontaktschicht (15) gezeigt. Die Wundauflage (10) besteht aus einer Trägerschicht (hier nicht sichtbar) aus einem wasserundurchlässigen und wasserdampfdurchlässigen Polyurethanfilm, die vollflächig mit einem Acrylatkleber beschichtet ist. Auf die Trägerschicht ist eine absorbierende hydrophile Polyurethanschaumschicht aufgebracht (hier nicht dargestellt), auf die eine Hydrogelmatrix als Wundkontaktschicht (15) aufgebracht ist. In die Wundkontaktschicht (15) sind eine Vielzahl an kreisförmigen Löchern (16) mit einem Durchmesser von 4,5 mm eingebracht, um den Durchfluss von Wundexsudat von der Wunde in die absorbierende Schicht zu ermöglichen. Durch die Wundkontaktschicht (15) wird ein Einwachsen von neu gebildeten Zellen in die Poren des Polyurethanschaums verhindert. Weiterhin vermittelt die Wundkontaktschicht (15) eine Klebrigkeit (Tack) auf der Wundstelle und der umgebenden Haut, wodurch ein Anlegen des Verbandes erleichtert wird. Gleichzeitig weist die Wundkontaktschicht (15) einen die Wundheilung fördernden pH-Wert auf. Durch die Löcher (16) kann aber auch das in dem hydrophilen Polyurethanschaum enthaltene Wasser an die Wunde abgegeben werden. Die mehrschichtige Wundauflage (10) kann auch einen Kleberand aufweisen, d.h. als so genannter Inselverband gefertigt werden (hier nicht dargestellt).

Mit Figur 2 ist eine weitere Ausführungsform einer erfindungsgemäßen Wundauflage gezeigt. Die Wundauflage (20) umfasst eine mit einer absorbierenden Schicht (23) kongruente Trägerschicht (21) aus einem wasserundurchlässigen und wasserdampfdurchlässigen Polyurethanschaum. Die Wundauflage (20) weist weiterhin zwischen der absorbierenden Schicht (23) und der Trägerschicht (21) eine erste wasserhaltige Hydrogelmatrix (22) auf. Die Wasserhaltige Hydrogelmatrix dient sowohl zur Fixierung der absorbierenden Schicht an die Trägerschicht als auch als zusätzlicher Wasserspeicher für die Wundauflage. Die Wundauflage umfasst eine absorbierende Schicht (23) mit einer Schichtdicke von 2,8 mm, eine Trägerschicht (21) mit einer Schichtdicke von 1,5 mm und eine erste Hydrogelmatrix (22) mit einer Schichtdicke von 0,8 mm. Die absorbierende Schicht (23) wird aus einem offenporigen hydrophilen Polyurethanschaum gebildet, der eine Porengröße von durchschnittlich 208 $\mu$m aufweist. Der Polyurethanschaum umfasst dabei einen Wasseranteil von mindestens 10 Gew.-%. Auf die erste Seite des Polyurethanschaums ist eine zweite Hydrogelmatrix als Wundkontaktschicht (25) aufgebracht. Die Hydrogelmatrix ist mit zylindrischen, im Querschnitt (parallel zur Wunde) kreisförmigen Kanälen (26) ausgestattet, so dass ein verbesserter Wundexsudatfluss von der Wunde in den absorbierenden hydrophilen Schaum erfolgen kann. Bei der Herstellung der Wundauflage ist die noch viskose Hydrogelmatrix geringfügig in den Polyurethanschaum eingedrungen, so dass zwischen der Hydrogelmatrix und dem hydrophilen Polyurethanschaum eine Übergangsschicht (24) ausgebildet ist, die aus der Hydrogelmatrix und dem hydrophilen Polyurethanschaum besteht. Die Übergangsschicht ihrerseits weist Kanäle (27) auf, die nur mit Polyurethanschaum gefüllt sind und die kongruent zu den Kanälen in der Hydrogelmatrix angeordnet sind. Der Polyurethanschaum umfasst eine erste Seite mit einer Fläche von 25 cm$^2$, wobei die Kanäle (26) insgesamt eine Fläche von ca. 11 cm$^2$ einnehmen.

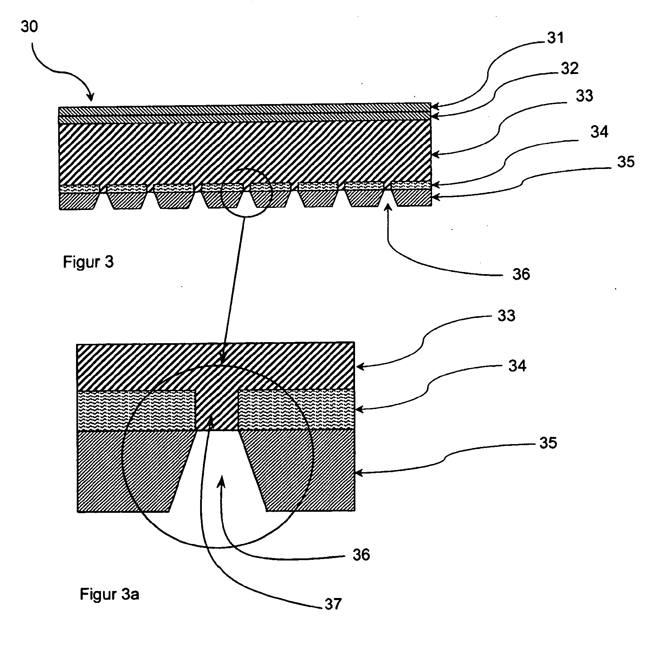

Mit Figur 3 ist eine dritte Ausführung einer erfindungsgemäßen Wundauflage gezeigt. Die Wundauflage (30) umfasst eine Trägerschicht (31) aus einem wasserundurchlässigen und wasserdampfdurchlässigen Polyurethanfilm, eine absorbierende Schicht (33) aus einem offenporigen hydrophilen Polyurethanschaum mit einem Wasseranteil von mindestens 10 Gew.-% (bezogen auf den Polyurethanschaum) und einer Wundkontaktschicht (35) aus einer wasserhaltigen Hydrogelmatrix mit einem Wasseranteil von ca. 45 Gew.-% (bezogen auf das Hydrogel). Die Trägerschicht (31) ist vollflächig mittels einem auf den Polymerfilm aufgetragenem Acrylatkleber (32) auf den hydrophilen Polymerschaum auflaminiert. Auf der im Anwendungsfall zur Wunde weisenden ersten Seite der absorbierenden Schicht ist eine wasserhaltige Hydrogelmatrix (35) aufgebracht, die ein Polyurethan-Polyharnstoff-Copolymer umfasst. Die Hydrogelmatrix ist mit konischen, im Querschnitt (parallel zur Wunde) kreisförmigen Kanälen (36) ausgestattet, so dass ein verbesserter Wundexsudatfluss von der Wunde in den absorbierenden hydrophilen Schaum erfolgen kann (vgl. Figur 3a). Bei der Herstellung der Wundauflage ist die noch viskose Hydrogelmatrix geringfügig in den Polyurethanschaum eingedrungen, so dass zwischen der Hydrogelmatrix und dem hydrophilen Polyurethanschaum eine Übergangsschicht (34) ausgebildet ist, die aus der Hydrogelmatrix und dem hydrophilen Polyurethanschaum besteht. Die Übergangsschicht ihrerseits weist Kanäle (37) auf, die nur mit Polyurethanschaum gefüllt sind und die kongruent zu den Kanälen in der Hydrogelmatrix angeordnet sind.

**Ausführungsbeispiel**

    A) bis D) Vergleichsbeispiele
    A) Herstellung des Hydrogels (Vergleichsbeispiel)

Zur Herstellung des Hydrogels werden folgende wässrigen Lösungen und Komponenten (Komponente A, B, C) eingesetzt:
Komponente A

| Propylenglycol USP30 (99,8 %) | Fa. Hedinger Auq GmbH; Stuttgart, Deutschland | 23,24 Gew.-% |
|---|---|---|
| Aqua purificata | Wasseraufbereitunqsanlage | 75,41 Gew.-% |
| NaCl, reinst, USP | Fa. Hedinqer Aug. GmbH; Stuttgart, Deutschland | 1,35 Gew.-% |

Zur Herstellung der Komponente A werden die Inhaltstoffe zusammengegeben und gerührt bis das Salz vollständig gelöst ist. Die Komponente A wird auf 2 °C abgekühlt.

Komponente B

| Jeffamin ED-2003 | Fa. Huntsman; Everberg, Belgien | 52,5 Gew.-% |
|---|---|---|
| Aqua purificata | Wasseraufbereitunqsanlage | 47,5 Gew.-% |

Zur Herstellung der wässrigen Komponente B wird das feste Jeffamin bei 50°C aufgeschmolzen und dem vorgelegten Wasser unter Rühren zugegeben. Die Komponente B wird auf Raumtemperatur abgekühlt.

Komponente C

| Aquapol PI-13000-3 | Fa. Carpenter; Richmond, USA | 100,0 Gew.-% |
|---|---|---|

Die Komponente C wird auf Raumtemperatur gebracht.

Die vorbereiteten Komponenten A, B, und C werden im Verhältnis 75,4:14,0:10,6 miteinander homogen durch homogenisieren mittels eines rotierenden Mischsystems vermischt und in die bereitgestellten Formen möglichst blasenfrei gegossen.

Der Propylenglykol-Gehalt der Zusammensetzung beträgt 17,5 Gew.-%. Das Verhältnis der reaktiven Gruppen von Isocyanat zu den Amingruppen des Diamins in der Zusammensetzung beträgt 1,23.

B1) Verwendeter Polyurethanschaum

Es wird ein hydrophiler Polyurethanschaum (Polyurethanschaum MCF.03R; Fa. Corpura, - Etten Leur, Niederlande) verwendet. Der trockene hydrophile Polyurethanschaum weist folgende Charakteristika auf:

a) Dichte: 77,9 - 83,7 kg/ m$^3$ (EN-ISO 845)
b) mittlere Porengröße: 208 $\mu$m (die Bestimmung erfolgte mikroskopisch, wobei ein Probenquerschnitt mikroskopiert wurde; die angegebene Porengröße entspricht dem Mittelwert von 5 zufällig ausgewählten und ausgemessenen Poren pro Probe)
c) Schichtdicke: 2,8 mm (Dickenmessgerät mit 25 cm$^2$ Teller, 2 g/cm$^2$ Belastung, gemessen nach EN ISO 9073-2)
d) Wasserdampfdurchlässigkeit: MVTR (upright) = 3593 g/ m$^2$/ 24 h (gemessen nach DIN EN 13726-2)
e) Absorption: freie Absorption $A_1$ = 20,5 g/ g (gemessen nach DIN EN 13726-1)
f) Quellvermögen: $\Delta V_0$ = 89,7 %

Das Quellvermögen $\Delta V_0$ eines Polyurethanschaums beschreibt die Volumenänderung, die ein trockener Polyurethanschaum erfährt, nachdem er seine maximale Absorption erreicht hat. Zur Bestimmung des Quellvermögens $\Delta V_0$ werden die räumlichen Abmessungen eines Probenstücks des trockenen Polymerschaums und die räumlichen Abmessungen dieses Probenstücks nach vollständiger Absorption gemäß der freien Absorption nach DIN EN 13726-1 bestimmt. Zur Bestimmung der Dicke (Höhe) wird ein Dickenmessgerät mit 25 cm$^2$ Teller verwendet, wobei eine Belastung von 2 g/cm$^2$ gemäß EN ISO 9073-2 eingestellt wird. Die laterale Ausdehnung (Länge, Breite) wird mittels einer Schieblehre bestimmt, ohne dass das Probenstück deformiert wird. Zur Bestimmung der Ausdehnung wird das jeweilige Probenstück spannungsfrei auf eine glatte Oberfläche abgelegt. Die Volumenänderung nach Absorption entspricht dem Quellvermögen $\Delta V_0$ des trockenen Polyurethanschaums, wobei alle drei Raumrichtungen beachtet werden.

| | Probe 1 | Probe 2 | Probe 3 | Mittelwert | Änderung / mm (%) |
|---|---|---|---|---|---|
| Länge ($l_0$) / mg | 50,0 | 50,0 | 50,0 | 50,0 | - |
| Breite ($b_0$) / mm | 50,0 | 50,0 | 50,0 | 50,0 | - |
| Höhe ($h_0$) / mm | 2,80 | 2,81 | 2,81 | 2,81 | - |
| Länge ($l_2$) / mm | 60,2 | 61,9 | 61,3 | 61,1 | 61,1 mm (22,2 %) |
| Breite ($b_2$) / mm | 61,7 | 63,5 | 62,8 | 62,7 | 12,7 mm (25,4 %) |

(fortgesetzt)

|  | Probe 1 | Probe 2 | Probe 3 | Mittelwert | Änderung / mm (%) |
|---|---|---|---|---|---|
| Höhe ($h_2$) / mm | 3,47 | 3,47 | 3,49 | 3,48 | 0,67 mm (23,8 %) |

$$\Delta V_0 = \frac{V_2 - V_0}{V_0} \bullet 100\% = \frac{(l_2 \bullet b_2 \bullet h_2) - (l_0 \bullet b_0 \bullet h_0)}{(l_0 \bullet b_0 \bullet h_0)} \bullet 100\% = 89,7\%$$

wobei gilt: $V_0$ = das Volumen des Probenstücks vor Absorption (gemessen unter Normklimabedingungen (23°C, 50% relative Luftfeuchtigkeit), und $V_2$ = das Volumen des Probenstücks nach vollständiger Absorption ist

g) Retentionswert: R = 52,8 %

Der Retentionswert R beschreibt die Menge Wasser, die der Polyurethanschaum in seine Polyurethanmatrix maximal einbinden kann, wobei das Wasser, das in die Poren aufgenommen werden könnte unberücksichtigt bleibt. Der Retentionswert wird bestimmt, in dem ein Probenstück von 5 cm x 5 cm (unter Normklima gelagert) aus einem hydrophilen Polyurethanschaum mit einer Dicke von höchstens 5 mm ausgestanzt und dessen Gewicht unter Normklimabedingungen vermessen wird. Das Probenstück wird hiernach einer freien Absorption mit Wasser analog DIN EN 13726-1 unterworfen. Das Wasser, das von den Poren aufgenommen wurde, wird dem Probenstück mittels einer Rolle (Gewicht 5000 g, Durchmesser 10 cm, Breite 5 cm) herausgequetscht, in dem die Probe mehrmals zwischen frische Zellstofftücher gelegt und mit der Rolle überrollt wird. Dieser Vorgang wird solange wiederholt, bis keine Wasserabsorption in den Zellstofftüchern erkennbar ist. Zur Bestimmung des Retentionswertes R wird der Wasseranteil $W_{ww}$, der nach dem Absorbieren und dem Ausquetschen in dem Polyurethanschaum enthalten ist gemäß DIN EN 14079 gemessen und wie folgt berechnet.

|  | Probe 1 | Probe 2 | Mittelwert |
|---|---|---|---|
| Gewicht trocken ($W_{tt}$) | 0,57 g | 0,58 g | 0,58 g |
| Gewicht nach Absorption | 11,44 g | 11,75 g | 11,60 g |
| Gewicht nach dem Ausdrücken ($W_{gg}$) | 1,22 g | 1,24 g | 1,23 g |

Die Dicke der vermessenen Probenstücke beträgt 2,80 mm.

$$R = W_{ww} = \frac{W_{gg} - W_{tt}}{W_{gg}} \bullet 100\% = 52,8\%$$

(gemessen nach DIN EN 14079)

wobei gilt: $W_{ww}$ = das Gewicht des Wassers in Prozent, dass nach der Absorption und dem Ausquetschen in der Polyurethanschaum enthalten ist

$W_{tt}$ = das Gewicht des Probenstücks nach dem Trocknen und

$W_{gg}$ = das Gewicht des Probenstücks nach Absorption und nach Ausquetschen

B2) Konditionieren des Polyurethanschaums

Der trockene hydrophile Polyurethanschaum wird auf die Abmessungen 20 x 30 cm zugeschnitten und für 3 Minuten in Wasser getaucht, so dass der Polyurethanschaum seine maximale Absorption erreicht. Der Polyurethanschaum wird aus dem Wasser entfernt und vorsichtig von Hand ausgequetscht. Der Polyurethanschaum wird hiernach mehrmals zwischen trockene Zellstofftücher gelegt und mittels einer Walze ausgequetscht (Liniendruck 10 N/ cm), so lange bis keine Wasserabsorption in den Zellstofftüchern erkennbar ist. Somit kann ausgeschlossen werden, dass kein Wasser in den Poren des Schaums vorhanden ist.

Der wasserhaltige Polyurethanschaum weist folgende Charakteristika auf:

a) Wassergehalt: Der hydrophile Polyurethanschaum hat einen Wasseranteil $W_w$ = 52,8 Gew.-% (gemessen nach DIN EN 14079), der dem Retentionswert R des trockenen Polyurethanschaums entspricht.

b) Absorption: freie Absorption $A_2$ = 16,2 g/ g (gemessen nach DIN EN 13726-1)

c) Quellvermögen: $\Delta V_1$ = 4 %

Das Quellvermögen des wasserhaltigen Polyurethanschaumstoffs wird in analoger Weise zu dem trockenen Polymerschaum bestimmt.

| | Probe 1 | Probe 2 | Probe 3 | Mittelwert | Änderung / mm (%) |
|---|---|---|---|---|---|
| Länge ($l_1$)/mg | 62,8 | 64,3 | 64,15 | 63,75 | - |
| Breite ($b_1$)/mm | 63,4 | 64,45 | 64,3 | 64,05 | - |
| Höhe ($h_1$)/mm | 3,4 | 3,38 | 3,39 | 3,39 | - |
| Länge ($l_2$) / mm | 60,2 | 61,9 | 61,3 | 61,1 | 2,65 mm (4,3%) |
| Breite ($b_2$) / mm | 61,7 | 63,5 | 62,8 | 62,7 | 1,35 mm (2,2%) |
| Höhe ($h_2$) / mm | 3,47 | 3,47 | 3,49 | 3,48 | -0,09 mm (-2,5%) |

$$\Delta V_1 = \frac{V_2 - V_1}{V_1} \bullet 100\% = \frac{(l_2 \bullet b_2 \bullet h_2) - (l_1 \bullet b_1 \bullet h_1)}{(l_1 \bullet b_1 \bullet h_1)} \bullet 100\% = 4\ \%$$

wobei gilt: $V_1$ = das Volumen des wasserhaltigen Probenstücks und

$V_2$ = das Volumen des Probenstücks nach vollständiger Absorption ist

C) Weitere verwendete Materialien

Als Trägerschicht wird ein 60 $\mu$m dicker, wasserundurchlässiger Polyurethanfilm (Fa. Exopack, Wrexham, United Kingdom) verwendet. Dieser Film ist mit einer Schichtdicke von 30 $\mu$m mit einem Pressure Sensitive Adhesive auf Acrylatbasis beschichtet. Der Film weist eine Wasserdampfdurchlässigkeit MVTR (upright) von 1100 g/ m$^2$/ 24 h (DIN EN 13726-1) auf.

D) Herstellung der Wundauflagen

Die Wundauflagen (Muster) werden von Hand nach folgendem Ablauf gefertigt.

1. Der Polyurethanschaum wird gemäß B) vorkonditioniert und bereitgestellt.

2. Zur Herstellung einer Hydrogelmatrix mit Kanälen wird eine PTFE-Form mit Noppenstruktur bereitgestellt. Die Noppen der Noppenstruktur sind kegelförmig und haben einen mittleren Durchmesser von 1,38 mm (Sockel 1,56 mm, Spitze 1,2 mm). Die Noppen haben eine Höhe von 1,35 mm und stehen in einem Rechteckraster im Abstand von 5 mm.

3. Das Hydrogel wird gemäß A) hergestellt und bereitgestellt, wobei das Hydrogel nach dem durchmischen und homogenisieren sofort weiterverarbeitet werden muss. Hierzu wird das Hydrogel zur Ausbildung der Hydrogelmatrix in die bereitgestellten Formen möglichst blasenfrei gegossen.

4. Das Gel wird mit einer PTFE-Rakel so verteilt, dass die Gelschicht die Höhe der Noppen (1,35 mm) aufweist. Überschüssiges Gel wird aus der Form entfernt.

5. Nach ca. 3 Minuten wird der vorkonditionierte Polyurethanschaum auf die Geloberfläche gelegt. Der Schaum wird mit einem Druck von 200 N/ m$^2$ angedrückt und gehalten.

6. Nach weiteren ca. 7 Minuten hat sich das Gel mit dem Schaum verbunden, so dass das Laminat aus wasserhaltigem Polyurethanschaum und wasserhaltigen Hydrogelmatrix aus der Form entfernt werden kann. Es wurde eine 0,3 mm dicke Übergangsschicht gebildet, die aus wasserhaltiger Hydrogelmatrix und wasserhaltigem Polyurethanschaum besteht.

7. Das Laminat wird mit der Hydrogelseite auf die vorbereitete Release-Folie gelegt (die silikonisierte Seite ist zum Gel gerichtet), so dass die zur Wunde gerichtete Seite geschützt ist.

8. Der Verbund wird auf der Schaumseite mit einem selbstklebenden Polyurethanfilm (vgl. D) abgedeckt, wobei der Polyurethanfilm mit einem Druck von 200 N/ m$^2$ fest angedrückt wird.

9. Aus dem mehrlagigen Materialverbund werden Wundauflagen mit einer Kantenlänge von 10 x 10 cm ausgestanzt.

Die somit hergestellte Wundauflage weist den mit Figur 3 beschriebenen Aufbau auf wobei mit Figur 3 kein Releaseliner gezeigt ist. Die Wundauflage besteht somit aus einem Laminat aus einer flexiblen, wasserhaltigen Hydrogelmatrix als Wundkontaktschicht, die 63,5 Gew.-% Wasser (bezogen auf die Hydrogelmatrix) enthält und einer absorbierende Schicht aus einem offenporigen hydrophilen Polyurethanschaum mit einem Wasseranteil von 52,8 Gew.-% (bezogen auf den Polyurethanschaum).

Des Weiteren weist die Wundauflage folgende Charakteristika auf:

a) Flächengewicht: 1550 g/ m$^2$ (gemessen nach DIN EN 29073-1)

b) Absorption: freie Absorption $A_1$ = 56 g/ 100 cm$^2$ (gemessen nach DIN EN 13726-1)

c) Wassergehalt insgesamt:

$$W_w = \frac{W_g - W_t}{W_g} \bullet 100\% = 58{,}8 \text{ Gew-\%}$$

d) Quellvermögen: $\Delta V$= 10 % (bestimmt gemäß oben beschriebener Methode)

Die vorliegende Wundauflage weist somit einen hohen Wasseranteil und eine hohe Absorption und eine geringe Quellung auf. Die Wundauflage ist somit bestens geeignet um in den Wundheilungsphasen 2 und 3 (Granulationsphase und Epithelisierungsphase) eingesetzt zu werden.

Die Wundauflage haftet nicht auf der Haut. Der Tack der Wundauflage wird mit dem Wert 1 beurteilt. Die Wundauflage muss beim Anlegen des Verbandes bis zur Befestigung, beispielsweise durch einen Fixierverband oder durch ein Klebepflaster, mit der Hand an der Wundstelle gehalten werden. Es kann daher vorteilhaft sein, dass die Wundauflage einen Kleberand aufweist, d.h. sie kann als so genannter Inselverband ausgebildet sein.

E) Herstellung eines Hydrogels

Unter Beibehaltung der vorstehend unter A) dargestellten Vorgehensweise werden weitere Hydrogelmatrices mit den folgenden, von A) abweichenden Zusammensetzungen hergestellt. Alle Angaben sind in Gew.-% bezogen auf die Zusammensetzung.

| Versuch | Isocyanat [1] | Diamin [2] | Polyharnstoff [3] | NCO /NH2 [4] | Propylenglykol | Wasser | NaCl |
|---------|--------------|-----------|-------------------|--------------|----------------|--------|------|
| 1.10 | 10,5 | 6,5 | 17,0 | 1,30 | 60,0 | 22,1 | 0,9 |
| 2.1.5 | 9,27 | 5,73 | 15,0 | 1,30 | 40,0 | 44,1 | 0,9 |
| 2.1.11 | 10,3 | 7,2 | 17,5 | 1,15 | 40,0 | 41,5 | 0,9 |
| [1] Isocyanat Vorpolymer (Aquapol PI-13000-3), Anteil der reaktiven Gruppen (NCO) 3,223 % | | | | | | | |
| [2] Diamin (Jeffamine ED-2003, Molekulargewicht 2.000), Amin-Gehalt 0,9554 mol/g | | | | | | | |
| [3] Summe aus Isocyanat und Diamin | | | | | | | |
| [4] Verhältnis der reaktiven Gruppen von Isocyanat zu den Amingruppen des Diamins | | | | | | | |

F) Herstellung einer mehrschichtigen Wundauflage

Zur Herstellung einer erfindungsgemäßen Wundauflage wird als erste Schicht eine Hydrogel-Zusammensetzung, wie vorstehend unter E) dargestellt, verwendet. Des weiteren wird als zweite Schicht ein Polyurethanschaum, wie vorstehend unter B1) dargestellt, verwendet. Die Wundauflage umfasst weiterhin die untere C) beschriebene Trägerschicht. Das Hydrogel weist nach dem Gießen eine Dicke von jeweils 1,0 mm auf. Nach dem Laminieren des Hydrogels mit dem Polyurethanschaum beträgt die Dicke des Hydrogels nur noch 0,8 mm. Zwischen Hydrogel und Polyurethanschaum wird durch das Laminieren eine weitere hybride Schicht ("Übergangsschicht") gebildet, welche aus wasserhaltigem Polyurethanschaum und wasserhaltiger Hydrogelmatrix besteht. Die Übergangsschicht entsteht, indem das noch nicht vollständig ausgehärtete Gel partiell in die Öffnungen des Schaums einwandert. Die weitere hybride Schicht, welche eine Dicke von ca. 0,3 mm aufweist, bildet sich nur an den Stellen der Polyurethanschaum-Schicht, welche in Kontakt mit dem Hydrogel stehen. Im Bereich der in das Hydrogel eingebrachten Öffnungen wird keine weitere hybride Schicht gebildet. Die Ausbildung einer weiteren hybriden Schicht, welche sich zwischen der ersten Schicht und der zweiten Schicht bildet, ist erwünscht, da die weitere hybride Schicht die Stabilität des Laminates erhöht.

Die Wundauflagen (Muster) werden von Hand nach folgendem Ablauf gefertigt:

1. Zur Herstellung einer Hydrogelmatrix mit Kanälen wird eine PTFE-Form (Teflon) mit Noppenstruktur bereitgestellt. Die Noppen der Noppenstruktur weisen eine Höhe von 1,0 mm auf und sind kegelförmig. Der mittlere Durchmesser der Noppen beträgt 4,5 mm (Sockel 5,1 mm, Spitze 3,9 mm). Der Abstand der Mittelpunkte der Noppen einer Reihe beträgt dabei 6,5 mm, so dass das Hydrogel nach dem Gießen Gel-Stege mit einer Breite von 2 mm aufweist. Der Abstand der Reihen zueinander ist mit 5,63 mm so gewählt, dass zwischen den Mittelpunkten von drei Löchern ein gleichseitiges Dreieck entsteht. Die Reihen weisen demnach eine zueinender versetzte Anordnung auf. Die Anordnung gewährleistet eine homogene Verteilung der offenen Fläche des Hydrogels. Die Fläche der Öffnungen im Hydrogel beträgt dabei wundseitig ca. 43,5%.

2. Die Hydrogel-Zusammensetzung wird gemäß E) hergestellt, wobei das Hydrogel nach dem Durchmischen und Homogenisieren sofort weiterverarbeitet werden muss. Hierzu wird das Hydrogel zur Ausbildung der Hydrogelmatrix in die unter 1) genannte Form möglichst blasenfrei gegossen.

3. Das Gel wird mit einer PTFE-Rakel so verteilt, dass die Gelschicht die Höhe der Noppen aufweist, also ca. 1 mm. Überschüssiges Gel wird aus der Form entfernt.

4. Nach ca. 3 Minuten wird der Polyurethanschaum auf die Geloberfläche gelegt, bevor das Hydrogel vollständig erhärtet ist. Der Schaum wird mit einem Druck von 200 N/ m$^2$ angedrückt und gehalten.

5. Nach ca. 7 Minuten hat sich das Gel mit dem Schaum verbunden, so dass das Laminat aus wasserhaltigem Polyurethanschaum und wasserhaltigen Hydrogelmatrix aus der Form entfernt werden kann. An der Kontaktfläche von Hydrogel und Polyurethanschaum wurde eine ca. 0,3 mm dicke Übergangsschicht gebildet, die aus wasserhaltiger Hydrogelmatrix und wasserhaltigem Polyurethanschaum besteht.

6. Das Laminat wird mit der Hydrogelseite auf die vorbereitete silikonisierte Release-Folie gelegt (die silikonisierte Seite ist zum Gel gerichtet). Die Release-Folie dient dem Schutz der Wundkontaktschicht.

7. Der Verbund wird auf der Polyurethanschaumseite mit einem selbstklebenden Polyurethanfilm (vgl. C) abgedeckt, wobei der Polyurethanfilm mit einem Druck von 200 N/ m$^2$ mindestens 10 Sekunden fest angedrückt wird.

8. Aus dem mehrlagigen Materialverbund werden Wundauflagen mit einer Kantenlänge von 10 x 10 cm ausgestanzt.

Im Unterschied zu dem unter D) beschriebenem Vorgehen, wurde auf eine Vorkonditionierung des Polyurethanschaumes, d.h. auf Schritt 1 von D), verzichtet

Die somit hergestellte Wundauflage weist den mit Figur 3 beschriebenen Aufbau auf, wobei mit Figur 3 kein Releaseliner gezeigt ist.

G) Messmethoden

Die Absorptionsfähigkeit für demineralisiertes Wasser wird in Anlehnung an EN13726-1 bestimmt. Die Messung erfolgt bei 37°C. Es werden 2,5 x 2,5 cm große Probenstücke aus der Mitte der Hydrogelschicht geschnitten. Eine ggf. vorhandene Abdeckfolie wird abgezogen und entfernt. Die Proben werden in ein Becherglas eingewogen. Anschließend wird die 40-fache Menge an demineralisiertem Wasser zugeben. Das Becherglas wird mit einem Uhrglas abgedeckt. Nach 48h werden die Proben erneut gewogen. Die Wasseraufnahme wird in g Wasser pro g Gelstück berechnet (g/g).

Der pH-Wert einer Wundauflage wird im Zusammenhang mit der vorliegenden Erfindung durch Einlegen des Laminats aus Hydrogel und Polyurethanschaum (ohne Trägerschicht und Releasefolie) in Wasser und Messung des pH-Wertes der Lösung bestimmt. Die Messung erfolgt bei Raumtemperatur (20 °C). Es werden 2,5 x 2,5 cm große Probenstücke aus der Mitte der Hydrogelschicht geschnitten. Eine ggf. vorhandene Abdeckfolie wird abgezogen und entfernt. Die Proben werden in ein Becherglas eingewogen. Anschließend 12,5ml demineralisiertem Wasser zugeben. Das Becherglas wird mit einem Uhrglas abgedeckt. Nach einem Zeitraum von 24 h wird die Probe aus der Lösung entfernt. Danach wird eine pH-Elektrode in die auf 20 °C temperiert Lösung getaucht. Eine Ablesung des pH-Wertes erfolgt, sobald der angezeigte Zahlenwert stabil bleibt.

Die Klebrigkeit (Tack) der Wundauflage auf unversehrter Haut wurde abgeschätzt. Auf dem Markt erhältliche Produkte wurden dabei von einer Person in 5 Kategorien eingeteilt. Ein Tack von 1 bedeutet dabei, dass die Wundauflage nicht auf gesunder Haut klebt, während ein Tack von 5 eine sehr hohe Klebrigkeit bedeutet. Die Bewertung erfolgte anhand des subjektiven Eindrucks einer Person anhand der Klebrigkeit der hergestellten Wundauflagen zwischen Zeigefinder und Gel.

H) Eigenschaften der Wundauflagen

|  | Tack | pH | Absorption |
|---|---|---|---|
| Wundauflage [1]) mit Hydrogel aus Versuch 1.10 | 5 | 8,83 | 8,63 g/g |
| Wundauflage [1]) mit Hydrogel aus Versuch 2.1.5 | 4 | 7,44 | 8,55 g/g |
| Wundauflage [1]) mit Hydrogel aus Versuch 2.1.11 | 5 | 8,18 | 9,98 g/g |
| Wundauflage [2]) aus Versuch A bis C) | 1 | nicht gemessen | |

[1]) Hydrogel Dicke 0,8 mm; Polyurethanschaum Dicke 2,8 mm
[2]) Hydrogel Dicke 1,35 mm; Polyurethanschaum Dicke 2,8 mm
[3]) Absorption von demineralisiertem Wasser nach 48 h bezogen auf Eigengewicht (g/g)

Die Wundauflagen weisen einen hohen Wasseranteil, eine hohe Absorption und eine geringe Quellung auf.

Die Wundauflage mit dem Hydrogel aus Versuch 1.10 weist einen unerwünscht hohen pH-Wert von 8,83 auf, der für die Wundheilung nicht förderlich ist. Weiterhin weist die Wundauflage einen Tack von 5 auf, d.h. die Wundauflage haftet sehr gut auf der Haut, lässt sich aber nicht völlig schmerzfrei entfernen. Der sehr starke Tack von 5 könnte beim Verbandswechsel insbesondere die empfindlichen Wundränder beschädigen.

Die Wundauflage mit dem Hydrogel aus Versuch 2.1.5 weist einen sehr vorteilhaften, die Wundheilung unterstützenden pH-Wert von 7,44 auf. Weiterhin weist die Wundauflage einen Tack von 4 auf, d.h. die Wundauflage haftet gut auf der Haut und lässt sich leicht entfernen. Der Tack liegt im erwünschten Bereich.

Die Wundauflage mit dem Hydrogel aus Versuch 2.1.11 weist einen unerwünscht hohen pH-Wert von pH-Wert von 8,18 auf. Weiterhin weist die Wundauflage einen Tack von 5 auf, d.h. die Wundauflage haftet sehr gut auf der Haut, lässt sich aber nicht völlig schmerzfrei entfernen. Der sehr starke Tack von 5 könnte beim Verbandswechsel insbesondere die empfindlichen Wundränder beschädigen.

Die nach A) bis C) hergestellte Wundauflage klebt nicht auf der Haut (Tack 1).

**Patentansprüche**

1. Mehrschichtige Wundauflage umfassend

    a) eine erste Schicht als Wundkontaktschicht, die eine wasserhaltige Hydrogelmatrix umfasst, und
    b) mindestens eine zweite absorbierende Schicht,

    **dadurch gekennzeichnet, dass** die Hydrogelmatrix 37 bis 43 Gew.-% Propylenglykol, ein Vorpolymer mit Isophorondiisocyanatendgruppen und ein Diamin auf Polyethylenoxidbasis in einer Menge von insgesamt 12 bis 16,5 Gew.-%, 0 bis 5 Gew.-% eines anorganischen Chlorids, sowie Rest Wasser umfasst, wobei das Verhältnis der reaktiven Gruppen von Isocyanat zu den Amingruppen des Diamins 1,25 bis 1,35 betragen soll.

2. Wundauflage nach Anspruch 1, wobei die Hydrogelmatrix 8 bis 10,5 Gew.-% eines Vorpolymers mit Isophorondiisocyanatendgruppen und 4 bis 7 Gew.-% eines Diamins auf Polyethylenoxidbasis umfasst.

3. Wundauflage nach mindestens einem der Ansprüche 1 oder 2, wobei die Hydrogelmatrix 39 bis 41 Gew.-% Propylenglykol und ein Vorpolymer mit Isophorondiisocyanatendgruppen und ein Diamin auf Polyethylenoxidbasis in einer Menge von insgesamt 14 bis 16 Gew.-% umfasst.

4. Wundauflage nach mindestens einem der vorangehenden Ansprüche, wobei die Hydrogelmatrix 40 Gew.-% Propylenglykol, 8,5 Gew.-% eines Vorpolymers mit Isophorondiisocyanatendgruppen und 6,5 Gew.-% eines Diamins auf Polyethylenoxidbasis umfasst.

5. Wundauflage nach mindestens einem der vorangehenden Ansprüche, wobei die zweite absorbierende Schicht einen hydrophilen Polyurethanschaum umfasst, wobei der Polyurethanschaum einen Wasseranteil von mindestens 10 Gew.-% Wasser und höchstens 80 Gew.-% Wasser, vorzugsweise mindestens 35 Gew.-% Wasser und höchstens 65 Gew.-% Wasser, aufweist.

6. Wundauflage nach Anspruch 5, **dadurch gekennzeichnet, dass** das Wasser homogen in dem Polyurethanschaum verteilt ist.

7. Wundauflage nach mindestens einem der vorhergehenden Ansprüche, **dadurch gekennzeichnet, dass** der trockene Polyurethanschaum eine Bruchdehnung nach ISO1798-M1 von 50 bis 105 kPa aufweist.

8. Wundauflage nach mindestens einem der vorhergehenden Ansprüche, **dadurch gekennzeichnet, dass** der Polyurethanschaum eine Dicke von 2,0 bis 5,5 mm aufweist.

9. Wundauflage nach mindestens einem der vorhergehenden Ansprüche, **dadurch gekennzeichnet, dass** die Wundkontaktschicht eine Vielzahl an Löchern, Kanälen oder Öffnungen aufweist, welche die Wundkontaktschicht vollständig durchdringen, so dass Flüssigkeit von außen durch die Wundkontaktschicht hindurch zur zweiten absorbierenden Schicht gelangen kann.

10. Wundauflage nach Anspruch 9, **dadurch gekennzeichnet, dass** die Löchern, Kanälen oder Öffnungen in einem regelmäßigem Muster vorliegen.

11. Wundauflage nach Anspruch 9 oder 10, **dadurch gekennzeichnet, dass** die Löchern, Kanäle oder Öffnungen 15 % bis 70 % der Fläche der Wundkontaktschicht einnehmen.

12. Wundauflage nach mindestens einem der vorhergehenden Ansprüche 9 bis 11, **dadurch gekennzeichnet, dass** die Wundkontaktschicht Löcher, Kanäle oder Öffnungen aufweist, die einen Durchmesser von jeweils 2 bis 6 mm aufweisen.

13. Wundauflage nach mindestens einem der vorhergehenden Ansprüche, **dadurch gekennzeichnet, dass** die Wundkontaktschicht eine Dicke von 0,6 bis 1,0 mm aufweist.

14. Wundauflage nach mindestens einem der vorhergehenden Ansprüche, **dadurch gekennzeichnet, dass** die Wundkontaktschicht weiterhin einen Polymerfilm, eine Hydrokolloidmatrix, ein Polymernetz, ein Nonwoven oder ein Adhäsiv umfasst.

15. Wundauflage nach mindestens einem der vorhergehenden Ansprüche, **dadurch gekennzeichnet, dass** die Wundauflage als Trägerschicht weiterhin einen wasserundurchlässigen und wasserdampfdurchlässigen Polymerfilm oder einen wasserundurchlässigen und wasserdampfdurchlässigen Polymerschaum umfasst.

16. Verfahren zur Herstellung einer mehrschichtigen Wundauflage gemäß einem der vorangehenden Ansprüche, wobei das Verfahren die Verfahrensschritte umfasst:

a) Bereitstellen eines hydrophilen Polyurethanschaums, wobei der Polyurethanschaum einen Wasseranteil von mindestens 10 Gew.-% Wasser und höchstens 80 Gew.-% Wasser aufweist.
b) Laminieren des unter a) hergestellten wasserhaltigen Polyurethanschaums auf eine Hydrogelmatrix, wobei die Hydrogelmatrix 37 bis 43 Gew. % Propylenglykol, ein Vorpolymer mit Isophorondiisocyanatendgruppen und ein Diamin auf Polyethylenoxidbasis in einer Menge von insgesamt 12 bis 16,5 Gew. %, 0 bis 5 Gew. % eines anorganischen Chlorids, sowie Rest Wasser umfasst, mit der Maßgabe, dass das Verhältnis der reaktiven Gruppen von Isocyanat zu den Amingruppen des Diamins 1,25 bis 1,35 betragen soll.

17. Hydrogelmatrix als Wundkontaktschicht, umfassend 37 bis 43 Gew. % Propylenglykol, ein Vorpolymer mit Isophorondiisocyanatendgruppen und ein Diamin auf Polyethylenoxidbasis in einer Menge von insgesamt 12 bis 16,5 Gew. %, 0 bis 5 Gew. % eines anorganischen Chlorids, sowie Rest Wasser umfasst, wobei das Verhältnis der reaktiven Gruppen von Isocyanat zu den Amingruppen des Diamins 1,25 bis 1,35 betragen soll, zur Behandlung von Gewebedefekten.

**Claims**

1. Multilayered wound dressing comprising

a) a first layer as a wound contact layer comprising a water-containing hydrogel matrix, and

b) at least one second absorbent layer,

**characterized in that** the hydrogel matrix comprises from 37 to 43 wt% of propylene glycol, altogether from 12 to 16.5 wt% of a prepolymer having isophorone diisocyanate end groups and of a diamine based on polyethylene oxide and from 0 to 5 wt% of an inorganic chloride, balance water, wherein the ratio for reactive groups of isocyanate to amine groups of diamine shall be in the range from 1.25 to 1.35.

2. Wound dressing according to Claim 1, wherein the hydrogel matrix comprises from 8 to 10.5 wt% of a prepolymer having isophorone diisocyanate end groups and from 4 to 7 wt% of a diamine based on polyethylene oxide.

3. Wound dressing according to either or both of Claims 1 and 2, wherein the hydrogel matrix comprises from 39 to 41 wt% of propylene glycol and altogether from 14 to 16 wt% of a prepolymer having isophorone diisocyanate end groups and of a diamine based on polyethylene oxide.

4. Wound dressing according to at least one preceding claim, wherein the hydrogel matrix comprises 40 wt% of propylene glycol, 8.5 wt% of a prepolymer having isophorone diisocyanate end groups and 6.5 wt% of a diamine based on polyethylene oxide.

5. Wound dressing according to at least one preceding claim, wherein the second absorbent layer comprises a hydrophilic polyurethane foam, wherein the polyurethane foam has a water content of at least 10 wt% of water and at most 80 wt% of water, preferably at least 35 wt% of water and at most 65 wt% of water.

6. Wound dressing according to Claim 5, **characterized in that** the water forms a homogeneous distribution in the polyurethane foam.

7. Wound dressing according to at least one preceding claim, **characterized in that** the dry polyurethane foam has an ISO1798-M1 elongation at break in the range from 50 to 105 kPa.

8. Wound dressing according to at least one preceding claim, **characterized in that** the polyurethane foam is from 2.0 to 5.5 mm in thickness.

9. Wound dressing according to at least one preceding claim, **characterized in that** the wound contact layer has a multiplicity of holes, channels or openings passing completely through the wound contact layer to allow liquid to pass from the outside through the wound contact layer to the second absorbent layer.

10. Wound dressing according to Claim 9, **characterized in that** the holes, channels or openings are in a regular pattern.

11. Wound dressing according to Claim 9 or 10, **characterized in that** the holes, channels or openings occupy from 15% to 70% of the area of the wound contact layer.

12. Wound dressing according to at least one of preceding Claims 9 to 11, **characterized in that** the wound contact layer has holes, channels or openings each from 2 to 6 mm in diameter.

13. Wound dressing according to at least one preceding claim, **characterized in that** the wound contact layer is from 0.6 to 1.0 mm in thickness.

14. Wound dressing according to at least one preceding claim, **characterized in that** the wound contact layer further comprises a polymer film, a hydrocolloid matrix, a polymer mesh, a nonwoven or an adhesive.

15. Wound dressing according to at least one preceding claim, **characterized in that** the wound dressing further comprises a water impermeable and moisture vapor permeable polymer film, or a water impermeable and moisture vapor permeable polymer foam, as a backing layer.

16. Process for producing a multilayered wound dressing according to any preceding claim, said process comprising the steps of:

a) providing a hydrophilic polyurethane foam, wherein the polyurethane foam has a water content of at least 10 wt% of water and at most 80 wt% of water,
b) laminating the water-containing polyurethane foam produced under a) onto a hydrogel matrix, wherein the hydrogel matrix comprises from 37 to 43 wt% of propylene glycol, altogether from 12 to 16.5 wt% of a prepolymer having isophorone diisocyanate end groups and of a diamine based on polyethylene oxide and from 0 to 5 wt% of an inorganic chloride, balance water, with the proviso that the ratio for reactive groups of isocyanate to amine groups of diamine shall be in the range from 1.25 to 1.35.

**17.** Hydrogel matrix useful as a wound contact layer and comprising from 37 to 43 wt% of propylene glycol, altogether from 12 to 16.5 wt% of a prepolymer having isophorone diisocyanate end groups and of a diamine based on polyethylene oxide and from 0 to 5 wt% of an inorganic chloride, balance water, wherein the ratio for reactive groups of isocyanate to amine groups of diamine shall be in the range from 1.25 to 1.35, for treatment of tissue defects.

## Revendications

**1.** Pansement à plusieurs couches, comprenant

a) une première couche comme couche de contact avec la plaie, qui comprend une matrice d'hydrogel contenant de l'eau, et
b) au moins une deuxième couche absorbante,

**caractérisé en ce que** la matrice d'hydrogel comprend 37 à 43% en poids de propylèneglycol, un prépolymère présentant des groupes terminaux isophoronediisocyanate et une diamine à base de poly(oxyde d'éthylène) en une quantité totale de 12 à 16,5% en poids, 0 à 5% en poids d'un chlorure inorganique ainsi que, pour le reste, de l'eau, le rapport des groupes réactifs isocyanate aux groupes amine de la diamine valant 1,25 à 1,35.

**2.** Pansement selon la revendication 1, la matrice d'hydrogel comprenant 8 à 10,5% en poids d'un prépolymère présentant des groupes terminaux isophoronediisocyanate et 4 à 7% en poids d'une diamine à base de poly(oxyde d'éthylène).

**3.** Pansement selon au moins l'une quelconque des revendications 1 ou 2, la matrice d'hydrogel comprenant 39 à 41% en poids de propylèneglycol et un prépolymère présentant des groupes terminaux isophoronediisocyanate et une diamine à base de poly(oxyde d'éthylène) en une quantité totale de 14 à 16% en poids.

**4.** Pansement selon au moins l'une quelconque des revendications précédentes, la matrice d'hydrogel comprenant 40% en poids de propylèneglycol, 8,5% en poids d'un prépolymère présentant des groupes terminaux isophoronediisocyanate et 6,5% en poids d'une diamine à base de poly(oxyde d'éthylène).

**5.** Pansement selon au moins l'une quelconque des revendications précédentes, la deuxième couche absorbante comprenant une mousse de polyuréthane hydrophile, la mousse de polyuréthane présentant une proportion d'eau d'au moins 10% en poids d'eau et d'au plus 80% en poids d'eau, de préférence d'au moins 35% en poids d'eau et d'au plus 65% en poids d'eau.

**6.** Pansement selon la revendication 5, **caractérisé en ce que** l'eau est répartie de manière homogène dans la mousse de polyuréthane.

**7.** Pansement selon au moins l'une quelconque des revendications précédentes, **caractérisé en ce que** la mousse de polyuréthane sèche présente un allongement à la rupture selon la norme ISO1798-M1 de 50 à 105 kPa.

**8.** Pansement selon au moins l'une quelconque des revendications précédentes, **caractérisé en ce que** la mousse de polyuréthane présente une épaisseur de 2,0 à 5,5 mm.

**9.** Pansement selon au moins l'une quelconque des revendications précédentes, **caractérisé en ce que** la couche de contact avec la plaie présente une multitude de trous, de canaux ou d'ouvertures, qui traversent complètement la couche de contact avec la plaie de manière telle que du liquide peut arriver à partir de l'extérieur à travers la couche de contact avec la plaie jusqu'à la deuxième couche absorbante.

**10.** Pansement selon la revendication 9, **caractérisé en ce que** les trous, les canaux ou les ouvertures sont disposées selon un motif régulier.

11. Pansement selon la revendication 9 ou 10, **caractérisé en ce que** les trous, les canaux ou les ouvertures occupent 15% à 70% de la surface de la couche de contact avec la plaie.

12. Pansement selon au moins l'une quelconque des revendications 9 à 11, **caractérisé en ce que** la couche de contact avec la plaie présente des trous, des canaux ou des ouvertures qui présentent un diamètre à chaque fois de 2 à 6 mm.

13. Pansement selon au moins l'une quelconque des revendications précédentes, **caractérisé en ce que** la couche de contact avec la plaie présente une épaisseur de 0,6 à 1,0 mm.

14. Pansement selon au moins l'une quelconque des revendications précédentes, **caractérisé en ce que** la couche de contact avec la plaie comprend en outre un film polymère, une matrice hydrocolloïdale, une toile polymère, un non-tissé ou un adhésif.

15. Pansement selon au moins l'une quelconque des revendications précédentes, **caractérisé en ce que** le pansement comprend en outre, comme couche support, un film polymère imperméable à l'eau et perméable à la vapeur d'eau ou une mousse polymère imperméable à l'eau et perméable à la vapeur d'eau.

16. Procédé pour la fabrication d'un pansement à plusieurs couches selon l'une quelconque des revendications précédentes, le procédé présentant les étapes de procédé suivantes :

   a) mise à disposition d'une mousse de polyuréthane hydrophile, la mousse de polyuréthane présentant une proportion d'eau d'au moins 10% en poids d'eau et d'au plus 80% en poids d'eau.
   b) stratification de la mousse de polyuréthane contenant de l'eau préparée au point a) sur une matrice d'hydrogel, la matrice d'hydrogel comprenant 37 à 43% en poids de propylèneglycol, un prépolymère présentant des groupes terminaux isophoronediisocyanate et une diamine à base de poly(oxyde d'éthylène) en une quantité totale de 12 à 16,5% en poids, 0 à 5% en poids d'un chlorure inorganique ainsi que, pour le reste, de l'eau, à condition que le rapport des groupes réactifs isocyanate aux groupes amine de la diamine vaille 1,25 à 1,35.

17. Matrice d'hydrogel comme couche de contact avec la plaie, comprenant 37 à 43% en poids de propylèneglycol, un prépolymère présentant des groupes terminaux isophoronediisocyanate et une diamine à base de poly(oxyde d'éthylène) en une quantité totale de 12 à 16,5% en poids, 0 à 5% en poids d'un chlorure inorganique ainsi que, pour le reste, de l'eau, le rapport des groupes réactifs isocyanate aux groupes amine de la diamine valant 1,25 à 1,35, destinée au traitement de défauts des tissus.

10

15
16

Figur 1

20

21
22
23
24
25

Figur 2

26

23

24

25

26

Figur 2a

27

30

31
32
33
34
35

Figur 3

36

33

34

35

36

Figur 3a

37

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 455324 B1
- EP 528091 B1
- EP 567704 B1
- EP 630629 B1
- EP 0426422 B1
- EP 457977 B1
- EP 486522 B1
- EP 541390 B1
- EP 541391 B1
- EP 570430 B1
- EP 665856 B1
- EP 691113 B1
- EP 693913 B1
- EP 1082146 B1
- EP 855921 B1
- EP 1156838 B1
- WO 0238097 A1
- WO 0247761 A1
- WO 03011352 A1
- WO 03086255 A1
- WO 2004052415 A1
- EP 1658865 A1
- DE 102008031183